# EUROPEAN PATENT SPECIFICATION

(11) **EP 4 373 554 B1**
(45) Date of publication and mention of the grant of the patent: **19.08.2026**
(21) Application number: 22844422.0
(22) Date of filing: 02.06.2022
(51) Int. Cl.: A61M 16/08

(54) **MAGNETIC CONNECTOR**
MAGNETISCHER VERBINDER
CONNECTEUR MAGNÉTIQUE

(30) Priority: 22.07.2021 AU 2021902251
(43) Date of publication of application: 29.05.2024
(73) Proprietor: ResMed Pty Ltd, Bella Vista, New South Wales 2153 (AU)
(72) Inventor: KHERA, Rahul, Bella Vista, New South Wales 2153 (AU); KIRKPATRICK, Ryan Michael, Bella Vista, New South Wales 2153 (AU); TRUSCOTT, Michael Kenneth, Bella Vista, New South Wales 2153 (AU)
(74) Representative: Vossius & Partner Patentanwälte Rechtsanwälte mbB
(86) International application number: PCT/AU2022/050543
(87) International publication number: WO 2023/000013

(56) References cited:
- US-A- 5 645 302
- US-B2- 11 040 158
- US-B2- 11 040 158
- US-B2- 7 341 060
- US-B2- 8 573 200

## Description

### 1 BACKGROUND OF THE TECHNOLOGY

### 1.1 FIELD OF THE TECHNOLOGY

The present technology relates to one or more of the screening, diagnosis, monitoring, treatment, prevention and amelioration of respiratory-related disorders. The present technology also relates to medical devices or apparatus, and their use.

### 1.2 DESCRIPTION OF THE RELATED ART

### 1.2.1 Human Respiratory System and its Disorders

The respiratory system of the body facilitates gas exchange. The nose and mouth form the entrance to the airways of a patient.

The airways include a series of branching tubes, which become narrower, shorter and more numerous as they penetrate deeper into the lung. The prime function of the lung is gas exchange, allowing oxygen to move from the inhaled air into the venous blood and carbon dioxide to move in the opposite direction. The trachea divides into right and left main bronchi, which further divide eventually into terminal bronchioles. The bronchi make up the conducting airways, and do not take part in gas exchange. Further divisions of the airways lead to the respiratory bronchioles, and eventually to the alveoli. The alveolated region of the lung is where the gas exchange takes place, and is referred to as the respiratory zone. See *"*Respiratory Physiology", by John B. West, Lippincott Williams & Wilkins, 9th edition published 2012.

### 1.2.2 Therapies

Various respiratory therapies, such as Continuous Positive Airway Pressure (CPAP) therapy, Non-invasive ventilation (NIV), Invasive ventilation (IV), and High Flow Therapy (HFT) have been used to treat one or more of the above respiratory disorders.

### 1.2.2.1 Respiratory pressure therapies

Respiratory pressure therapy is the application of a supply of air to an entrance to the airways at a controlled target pressure that is nominally positive with respect to atmosphere throughout the patient's breathing cycle (in contrast to negative pressure therapies such as the tank ventilator or cuirass).

Continuous Positive Airway Pressure (CPAP) therapy has been used to treat Obstructive Sleep Apnea (OSA). The mechanism of action is that continuous positive airway pressure acts as a pneumatic splint and may prevent upper airway occlusion, such as by pushing the soft palate and tongue forward and away from the posterior oropharyngeal wall. Treatment of OSA by CPAP therapy may be voluntary, and hence patients may elect not to comply with therapy if they find devices used to provide such therapy one or more of: uncomfortable, difficult to use, expensive and aesthetically unappealing.

Non-invasive ventilation (NIV) provides ventilatory support to a patient through the upper airways to assist the patient breathing and/or maintain adequate oxygen levels in the body by doing some or all of the work of breathing. The ventilatory support is provided via a non-invasive patient interface. NIV has been used to treat CSR and respiratory failure, in forms such as OHS, COPD, NMD and Chest Wall disorders. In some forms, the comfort and effectiveness of these therapies may be improved.

Invasive ventilation (IV) provides ventilatory support to patients that are no longer able to effectively breathe themselves and may be provided using a tracheostomy tube or endotracheal tube. In some forms, the comfort and effectiveness of these therapies may be improved.

### 1.2.2.2 Flow therapies

Not all respiratory therapies aim to deliver a prescribed therapeutic pressure. Some respiratory therapies aim to deliver a prescribed respiratory volume, by delivering an inspiratory flow rate profile over a targeted duration, possibly superimposed on a positive baseline pressure. In other cases, the interface to the patient's airways is 'open' (unsealed) and the respiratory therapy may only supplement the patient's own spontaneous breathing with a flow of conditioned or enriched gas. In one example, High Flow therapy (HFT) is the provision of a continuous, heated, humidified flow of air to an entrance to the airway through an unsealed or open patient interface at a "treatment flow rate" that may be held approximately constant throughout the respiratory cycle. The treatment flow rate is nominally set to exceed the patient's peak inspiratory flow rate. HFT has been used to treat OSA, CSR, respiratory failure, COPD, and other respiratory disorders. One mechanism of action is that the high flow rate of air at the airway entrance improves ventilation efficiency by flushing, or washing out, expired CO2 from the patient's anatomical deadspace. Hence, HFT is thus sometimes referred to as a deadspace therapy (DST). Other benefits may include the elevated warmth and humidification (possibly of benefit in secretion management) and the potential for modest elevation of airway pressures. As an alternative to constant flow rate, the treatment flow rate may follow a profile that varies over the respiratory cycle.

Another form of flow therapy is long-term oxygen therapy (LTOT) or supplemental oxygen therapy. Doctors may prescribe a continuous flow of oxygen enriched air at a specified oxygen concentration (from 21%, the oxygen fraction in ambient air, to 100%) at a specified flow rate (e.g., 1 litre per minute (LPM), 2 LPM, 3 LPM, etc.) to be delivered to the patient's airway.

### 1.2.3 Respiratory Therapy Systems

These respiratory therapies may be provided by a respiratory therapy system or device. Such systems and devices may also be used to screen, diagnose, or monitor a condition without treating it.

A respiratory therapy system may comprise a Respiratory Pressure Therapy Device (RPT device), an air circuit, a humidifier, a patient interface, an oxygen source, and data management.

Another form of therapy system is a mandibular repositioning device.

### 1.2.3.1 Patient Interface

A patient interface may be used to interface respiratory equipment to its wearer, for example by providing a flow of air to an entrance to the airways. The flow of air may be provided via a mask to the nose and/or mouth, a tube to the mouth or a tracheostomy tube to the trachea of a patient. Depending upon the therapy to be applied, the patient interface may form a seal, e.g., with a region of the patient's face, to facilitate the delivery of gas at a pressure at sufficient variance with ambient pressure to effect therapy, e.g., at a positive pressure of about 10 cmH₂O relative to ambient pressure. For other forms of therapy, such as the delivery of oxygen, the patient interface may not include a seal sufficient to facilitate delivery to the airways of a supply of gas at a positive pressure of about 10 cmH₂O. For flow therapies such as nasal HFT, the patient interface is configured to insufflate the nares but specifically to avoid a complete seal. One example of such a patient interface is a nasal cannula.

Certain other mask systems may be functionally unsuitable for the present field. For example, purely ornamental masks may be unable to maintain a suitable pressure. Mask systems used for underwater swimming or diving may be configured to guard against ingress of water from an external higher pressure, but not to maintain air internally at a higher pressure than ambient.

Certain masks may be clinically unfavourable for the present technology e.g. if they block airflow via the nose and only allow it via the mouth.

Certain masks may be uncomfortable or impractical for the present technology if they require a patient to insert a portion of a mask structure in their mouth to create and maintain a seal via their lips.

Certain masks may be impractical for use while sleeping, e.g. for sleeping while lying on one's side in bed with a head on a pillow.

The design of a patient interface presents a number of challenges. The face has a complex three-dimensional shape. The size and shape of noses and heads varies considerably between individuals. Since the head includes bone, cartilage and soft tissue, different regions of the face respond differently to mechanical forces. The jaw or mandible may move relative to other bones of the skull. The whole head may move during the course of a period of respiratory therapy.

As a consequence of these challenges, some masks suffer from being one or more of obtrusive, aesthetically undesirable, costly, poorly fitting, difficult to use, and uncomfortable especially when worn for long periods of time or when a patient is unfamiliar with a system. Wrongly sized masks can give rise to reduced compliance, reduced comfort and poorer patient outcomes. Masks designed solely for aviators, masks designed as part of personal protection equipment (e.g. filter masks), SCUBA masks, or for the administration of anaesthetics may be tolerable for their original application, but nevertheless such masks may be undesirably uncomfortable to be worn for extended periods of time, e.g., several hours. This discomfort may lead to a reduction in patient compliance with therapy. This is even more so if the mask is to be worn during sleep.

CPAP therapy is highly effective to treat certain respiratory disorders, provided patients comply with therapy. If a mask is uncomfortable, or difficult to use a patient may not comply with therapy. Since it is often recommended that a patient regularly wash their mask, if a mask is difficult to clean (e.g., difficult to assemble or disassemble), patients may not clean their mask and this may impact on patient compliance.

While a mask for other applications (e.g. aviators) may not be suitable for use in treating sleep disordered breathing, a mask designed for use in treating sleep disordered breathing may be suitable for other applications.

For these reasons, patient interfaces for delivery of CPAP during sleep form a distinct field.

### 1.2.3.1.1 Seal-forming structure

Patient interfaces may include a seal-forming structure. Since it is in direct contact with the patient's face, the shape and configuration of the seal-forming structure can have a direct impact the effectiveness and comfort of the patient interface.

A patient interface may be partly characterised according to the design intent of where the seal-forming structure is to engage with the face in use. In one form of patient interface, a seal-forming structure may comprise a first sub-portion to form a seal around the left naris and a second sub-portion to form a seal around the right naris. In one form of patient interface, a seal-forming structure may comprise a single element that surrounds both nares in use. Such single element may be designed to for example overlay an upper lip region and a nasal bridge region of a face. In one form of patient interface a seal-forming structure may comprise an element that surrounds a mouth region in use, e.g. by forming a seal on a lower lip region of a face. In one form of patient interface, a seal-forming structure may comprise a single element that surrounds both nares and a mouth region in use. These different types of patient interfaces may be known by a variety of names by their manufacturer including nasal masks, full-face masks, nasal pillows, nasal puffs and oro-nasal masks.

A seal-forming structure that may be effective in one region of a patient's face may be inappropriate in another region, e.g. because of the different shape, structure, variability and sensitivity regions of the patient's face. For example, a seal on swimming goggles that overlays a patient's forehead may not be appropriate to use on a patient's nose.

Certain seal-forming structures may be designed for mass manufacture such that one design fit and be comfortable and effective for a wide range of different face shapes and sizes. To the extent to which there is a mismatch between the shape of the patient's face, and the seal-forming structure of the mass-manufactured patient interface, one or both must adapt in order for a seal to form.

One type of seal-forming structure extends around the periphery of the patient interface, and is intended to seal against the patient's face when force is applied to the patient interface with the seal-forming structure in confronting engagement with the patient's face. The seal-forming structure may include an air or fluid filled cushion, or a moulded or formed surface of a resilient seal element made of an elastomer such as a rubber. With this type of seal-forming structure, if the fit is not adequate, there will be gaps between the seal-forming structure and the face, and additional force will be required to force the patient interface against the face in order to achieve a seal.

Another type of seal-forming structure incorporates a flap seal of thin material positioned about the periphery of the mask so as to provide a self-sealing action against the face of the patient when positive pressure is applied within the mask. Like the previous style of seal forming portion, if the match between the face and the mask is not good, additional force may be required to achieve a seal, or the mask may leak. Furthermore, if the shape of the seal-forming structure does not match that of the patient, it may crease or buckle in use, giving rise to leaks.

Another type of seal-forming structure may comprise a friction-fit element, e.g. for insertion into a naris, however some patients find these uncomfortable.

Another form of seal-forming structure may use adhesive to achieve a seal. Some patients may find it inconvenient to constantly apply and remove an adhesive to their face.

A range of patient interface seal-forming structure technologies are disclosed in the following patent applications, assigned to ResMed Limited: WO 1998/004,310; WO 2006/074,513; WO 2010/135,785.

One form of nasal pillow is found in the Adam Circuit manufactured by Puritan Bennett. Another nasal pillow, or nasal puff is the subject of US Patent 4,782,832 (Trimble et al.), assigned to Puritan-Bennett Corporation.

ResMed Limited has manufactured the following products that incorporate nasal pillows: SWIFTTM nasal pillows mask, SWIFTTM II nasal pillows mask, SWIFTTM LT nasal pillows mask, SWIFTTM FX nasal pillows mask and MIRAGE LIBERTYTM full-face mask. The following patent applications, assigned to ResMed Limited, describe examples of nasal pillows masks: International Patent Application WO2004/073,778 (describing amongst other things aspects of the ResMed Limited SWIFTTM nasal pillows), US Patent Application 2009/0044808 (describing amongst other things aspects of the ResMed Limited SWIFTTM LT nasal pillows); International Patent Applications WO 2005/063,328 and WO 2006/130,903 (describing amongst other things aspects of the ResMed Limited MIRAGE LIBERTYTM full-face mask); International Patent Application WO 2009/052,560 (describing amongst other things aspects of the ResMed Limited SWIFTTM FX nasal pillows).

### 1.2.3.1.2 Positioning and stabilising

A seal-forming structure of a patient interface used for positive air pressure therapy is subject to the corresponding force of the air pressure to disrupt a seal. Thus a variety of techniques have been used to position the seal-forming structure, and to maintain it in sealing relation with the appropriate portion of the face.

One technique is the use of adhesives. See for example US Patent Application Publication No. US 2010/0000534. However, the use of adhesives may be uncomfortable for some.

Another technique is the use of one or more straps and/or stabilising harnesses. Many such harnesses suffer from being one or more of ill-fitting, bulky, uncomfortable and awkward to use.

### 1.2.3.2 Respiratory Pressure Therapy (RPT) Device

A respiratory pressure therapy (RPT) device may be used individually or as part of a system to deliver one or more of a number of therapies described above, such as by operating the device to generate a flow of air for delivery to an interface to the airways. The flow of air may be pressure-controlled (for respiratory pressure therapies) or flow-controlled (for flow therapies such as HFT). Thus RPT devices may also act as flow therapy devices. Examples of RPT devices include a CPAP device and a ventilator.

The designer of a device may be presented with an infinite number of choices to make. Design criteria often conflict, meaning that certain design choices are far from routine or inevitable. Furthermore, the comfort and efficacy of certain aspects may be highly sensitive to small, subtle changes in one or more parameters.

### 1.2.3.3 Air circuit

An air circuit is a conduit or a tube constructed and arranged to allow, in use, a flow of air to travel between two components of a respiratory therapy system such as the RPT device and the patient interface. In some cases, there may be separate limbs of the air circuit for inhalation and exhalation. In other cases, a single limb air circuit is used for both inhalation and exhalation.

The presence of the air circuit may be a significant factor in a patient's lack of compliance with therapy. The air circuit connects the patient interface (which moves with the patient) to the RPT (which is typically stationary). This connection can lead to the patient feeling "tethered" to the RPT. This feeling may be alleviated if the air circuit can be connected to or disconnected from the patient interface quickly and easily (e.g. if the patient wishes to get up from the bed)

The connection between the air circuit and the patient interface is often of a snap-fit or interference type. Such connections require two parts of the connector to be pressed together to make the connection. While the force required to make the connection is usually not large in outright terms, it may result in the patient interface being pressed against the patient's face if the connection is made while the interface is being worn. This may be particularly problematic if the patent interface, in particular the portion of the patient interface surrounding the air circuit connection, is made from relatively soft materials. It would therefore be advantageous to develop a connector which can be quickly and easily connected and disconnected with the application of only a very low or substantially no axial force. Magnetic connector systems are disclosed for example in US 11040158 B2.

### 1.2.3.4 Humidifier

Delivery of a flow of air without humidification may cause drying of airways. The use of a humidifier with an RPT device and the patient interface produces humidified gas that minimizes drying of the nasal mucosa and increases patient airway comfort. In addition, in cooler climates, warm air applied generally to the face area in and about the patient interface is more comfortable than cold air.

### 1.2.3.5 Data Management

There may be clinical reasons to obtain data to determine whether the patient prescribed with respiratory therapy has been "compliant", e.g. that the patient has used their RPT device according to one or more "compliance rules". One example of a compliance rule for CPAP therapy is that a patient, in order to be deemed compliant, is required to use the RPT device for at least four hours a night for at least 21 of 30 consecutive days. In order to determine a patient's compliance, a provider of the RPT device, such as a health care provider, may manually obtain data describing the patient's therapy using the RPT device, calculate the usage over a predetermined time period, and compare with the compliance rule. Once the health care provider has determined that the patient has used their RPT device according to the compliance rule, the health care provider may notify a third party that the patient is compliant.

There may be other aspects of a patient's therapy that would benefit from communication of therapy data to a third party or external system.

Existing processes to communicate and manage such data can be one or more of costly, time-consuming, and error-prone.

### 1.2.3.6 Vent technologies

Some forms of treatment systems may include a vent to allow the washout of exhaled carbon dioxide. The vent may allow a flow of gas from an interior space of a patient interface, e.g., the plenum chamber, to an exterior of the patient interface, e.g., to ambient.

### 1.2.4 Screening, Diagnosis, and Monitoring Systems

Polysomnography (PSG) is a conventional system for diagnosis and monitoring of cardio-pulmonary disorders, and typically involves expert clinical staff to apply the system. PSG typically involves the placement of 15 to 20 contact sensors on a patient in order to record various bodily signals such as electroencephalography (EEG), electrocardiography (ECG), electrooculograpy (EOG), electromyography (EMG), etc. PSG for sleep disordered breathing has involved two nights of observation of a patient in a clinic, one night of pure diagnosis and a second night of titration of treatment parameters by a clinician. PSG is therefore expensive and inconvenient. In particular, it is unsuitable for home screening / diagnosis / monitoring of sleep disordered breathing.

Screening and diagnosis generally describe the identification of a condition from its signs and symptoms. Screening typically gives a true / false result indicating whether or not a patient's SDB is severe enough to warrant further investigation, while diagnosis may result in clinically actionable information. Screening and diagnosis tend to be one-off processes, whereas monitoring the progress of a condition can continue indefinitely. Some screening / diagnosis systems are suitable only for screening / diagnosis, whereas some may also be used for monitoring.

Clinical experts may be able to screen, diagnose, or monitor patients adequately based on visual observation of PSG signals. However, there are circumstances where a clinical expert may not be available, or a clinical expert may not be affordable. Different clinical experts may disagree on a patient's condition. In addition, a given clinical expert may apply a different standard at different times.

### 2 BRIEF SUMMARY OF THE TECHNOLOGY

The present technology is directed towards providing medical devices used in the screening, diagnosis, monitoring, amelioration, treatment, or prevention of respiratory disorders having one or more of improved comfort, cost, efficacy, ease of use and manufacturability.

A first aspect of the present technology relates to apparatus used in the screening, diagnosis, monitoring, amelioration, treatment or prevention of a respiratory disorder.

Another aspect of the present technology relates to methods used in the screening, diagnosis, monitoring, amelioration, treatment or prevention of a respiratory disorder.

An aspect of certain forms of the present technology is to provide methods and/or apparatus that improve the compliance of patients with respiratory therapy.

One form of the present technology comprises a fluid connector for coupling an air circuit to a respiratory therapy system component, the connector comprising a first connector part and a second connector part,
the first connector part comprising a first body, a first passage extending through the first body, and a cylindrical outer wall, the cylindrical outer wall comprising a groove which extends around the circumference of the first body,
the second connector part comprising a second body, a second passage extending through the second body, and a plurality of arms extending from a first end of the second body, each arm comprising an inwardly extending projecting portion,
wherein the arms are configured to allow the inwardly extending projecting portions to engage the groove,
wherein the groove comprises a plurality of contiguous substantially helical portions having alternating orientations, wherein the number of substantially helical portions is twice the number of arms,
wherein at least one of the first and second connector parts comprises a magnetic portion and the other of the first and second connector parts comprises a second magnetic portion and/or a ferromagnetic portion, wherein magnetic attraction between the magnetic portion and the second magnetic portion and/or ferromagnetic portion biases the first and second connector parts towards a fully connected configuration,
and wherein, when the first and second connector parts are in a fully connected configuration, rotation of one of the connector parts relative to the other connector part causes the first and second bodies to move away from each other.

In examples:
a) the first body has a first end and a second end, wherein an entrance to the first passage is provided at the first end; b) the magnetic attraction between the magnetic portions or the magnetic portion and the ferromagnetic portion is sufficient to move the first and second connector parts to the fully connected configuration when the inwardly extending projecting portions engage portions of the groove which are closest to the first end of the first body; c) the portions of the groove which are closest to the first end of the first body are open to the first end of the first body; d) the portions of the groove which are closest to the first end of the first body each comprise a ramped portion configured to urge the arms apart when the first connector part is disconnected from the second connector part; e) each arm is connected to the external wall of the second body by a respective mounting portion, wherein each mounting portion comprises a resilient living hinge; f) each arm is resiliently flexible; g) a front edge of the first body is bevelled to urge the arms radially outward as the first and second connector parts are brought into contact; h) the attractive force between the magnetic portions is no more than 10N when the first and second connector parts are in the fully engaged position; i) a tension force of at least 20N is required to separate the first and second connector parts when in the fully engaged position.

Another form of the present technology comprises a fluid connector for coupling an air circuit to a respiratory therapy system component, the fluid connector comprising a first connector part and a second connector part,
the first connector part comprising a passage comprising an tubular inner wall, the tubular inner wall provided with at least one spiral groove extending from a first end of the tubular inner wall, the first connector part further comprising a first radially extending wall extending radially outward from a first end of the passage,
the second connector part comprising an tubular outer wall configured to be received by the tubular inner wall, the tubular outer wall provided with at least one radially projecting portion, the second connector part further comprising a second radially extending wall extending radially outward from the tubular outer wall, the second connector part having a second passage therethrough,
wherein at least one of the first and second connector parts comprises a magnetic portion and the other of the first and second connector parts comprises a second magnetic portion and/or a ferromagnetic portion,
wherein, when the first and second parts are in a connected configuration, the tubular outer wall is received by the tubular inner wall, the or each radially projecting portion engages a respective one of the at least one spiral groove, and the first radially extending wall abuts the second radially extending wall, and
wherein, when in the connected configuration, a magnetic attractive force between the magnetic portion and the second magnetic portion and/or ferromagnetic portion biases the first and second radially extending walls toward each other.

In examples:
a) the or each spiral groove has a groove entrance, wherein, when the second connector part is positioned relative to the first connector part such that the or each radially projecting portion engages a respective groove entrance, the magnetic attractive force is sufficient to move the first and second connector parts to the connected configuration in the absence of any external force on the connector parts; b) when the second connector part is positioned relative to the first connector part such that the or each radially projecting portion engages a respective groove entrance, the magnetic attractive force is at least 2N, for example 4N; c) when in the connected configuration, the magnetic attractive force is at least 10N, for example around 20N; d) the at least one spiral groove comprises a plurality of spiral grooves, wherein the entrances to the spiral grooves are evenly circumferentially spaced apart; e) the second connector part comprises the same number of radially projecting portions as the number of spiral grooves provided to the first connector part, wherein the radially projecting portions are evenly circumferentially spaced apart; f) the width of each spiral groove entrance is greater than the width of the radially projecting portion; g) the distance between adjacent spiral groove entrances is no more than ½ the width of the radially projecting portion; h) the spiral grooves have a constant helix angle; i) a helix angle of the spiral grooves decreases toward the end distal to the entrance; j) the helix angle of a first portion of the spiral groove, adjacent the groove entrance, is substantially 90 degrees, and the helix angle of a second portion of the spiral groove, distal the groove entrance, is between 10 degrees and 0 degrees; k) the first and second connector portions can be separated from each other by rotating one of the connector portions relative to the other; l) one or both of the first and second radially extending walls comprises, or is formed from, a resilient material to form a seal; m) one of the first and second connector parts comprises a cuff configured for connection to an air circuit or respiratory therapy system component; n) the first connector part is engaged with a patient interface; o) an air circuit is rotatably coupled with the connector; and/or p) the second connector part comprises a first body part and a second body part which is rotatable relative to the first body part.

Another aspect of one form of the present technology is the a fluid connector for coupling an air circuit to a respiratory therapy system component, the fluid connector comprising a first connector part and a second connector part, wherein the first and second parts are drawn to a connected configuration by a magnetic attractive force and are separable by rotating one of the connector parts relative to the other connector part.

Another aspect of one form of the technology comprises a patient interface comprising a fluid connector for coupling the patient interface to an air circuit, the connector comprising a first connector part and a second connector part, wherein the first and second parts are drawn to a connected configuration by a magnetic attractive force and are separable by rotating one of the connector parts relative to the other connector part.

Another aspect of one form of the technology comprises a patient interface comprising a first connector part of a fluid connector for coupling the patient interface to an air circuit, the connector comprising the first connector part and a second connector part, wherein, in use, the first and second parts are drawn to a connected configuration by a magnetic attractive force and are separable by rotating one of the connector parts relative to the other connector part.

Another aspect of one form of the technology comprises a patient interface comprising a first connector part of a fluid connector for coupling the patient interface to an air circuit,
the first connector part comprising a passage comprising an tubular inner wall, the tubular inner wall provided with at least one spiral groove extending from a first end of the tubular inner wall, the first connector part further comprising a first radially extending wall extending radially outward from a first end of the passage,
the first connector part comprising a magnetic portion and/or a ferromagnetic portion,
the first connector part configured to engage, in use, a second connector part comprising an tubular outer wall configured to be received by the tubular inner wall, the tubular outer wall provided with at least one radially projecting portion, the second connector part further comprising a second radially extending wall extending radially outward from the tubular outer wall, the second connector part having a second passage therethrough, the second connector part comprising a magnetic portion and/or a ferromagnetic portion,
wherein, when in the connected configuration, a magnetic attractive force between the magnetic portion and the second magnetic portion and/or ferromagnetic portion biases the first and second radially extending walls toward each other.

Another aspect of one form of the present technology is a patient interface that is moulded or otherwise constructed with a perimeter shape which is complementary to that of an intended wearer.

An aspect of one form of the present technology is a method of manufacturing apparatus.

An aspect of certain forms of the present technology is a medical device that is easy to use, e.g. by a person who does not have medical training, by a person who has limited dexterity, vision or by a person with limited experience in using this type of medical device.

An aspect of one form of the present technology is a portable RPT device that may be carried by a person, e.g., around the home of the person.

An aspect of one form of the present technology is a patient interface that may be washed in a home of a patient, e.g., in soapy water, without requiring specialised cleaning equipment. An aspect of one form of the present technology is a humidifier tank that may be washed in a home of a patient, e.g., in soapy water, without requiring specialised cleaning equipment.

The methods, systems, devices and apparatus described may be implemented so as to improve the functionality of a processor, such as a processor of a specific purpose computer, respiratory monitor and/or a respiratory therapy apparatus. Moreover, the described methods, systems, devices and apparatus can provide improvements in the technological field of automated management, monitoring and/or treatment of respiratory conditions, including, for example, sleep disordered breathing.

Of course, portions of the aspects may form sub-aspects of the present technology. Also, various ones of the sub-aspects and/or aspects may be combined in various manners and also constitute additional aspects or sub-aspects of the present technology.

Other features of the technology will be apparent from consideration of the information contained in the following detailed description, abstract, drawings and claims.

### 3 BRIEF DESCRIPTION OF THE DRAWINGS

The present technology is illustrated by way of example, and not by way of limitation, in the figures of the accompanying drawings, in which like reference numerals refer to similar elements including:

### 3.1 RESPIRATORY THERAPY SYSTEMS

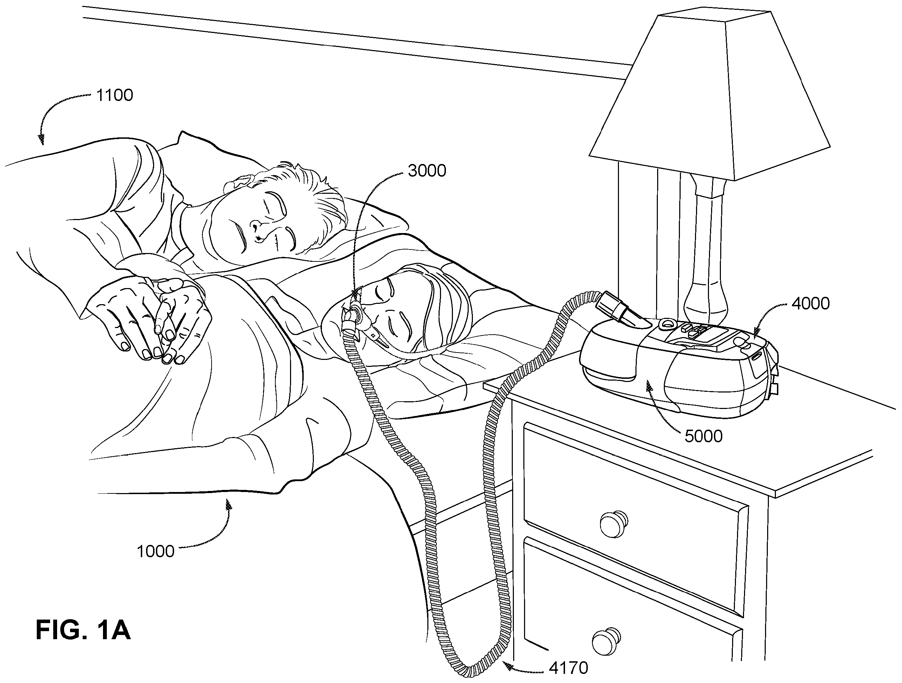

Fig. 1A shows a system including a patient 1000 wearing a patient interface 3000, in the form of nasal pillows, receiving a supply of air at positive pressure from an RPT device 4000. Air from the RPT device 4000 is humidified in a humidifier 5000, and passes along an air circuit 4170 to the patient 1000. A bed partner 1100 is also shown. The patient is sleeping in a supine sleeping position.

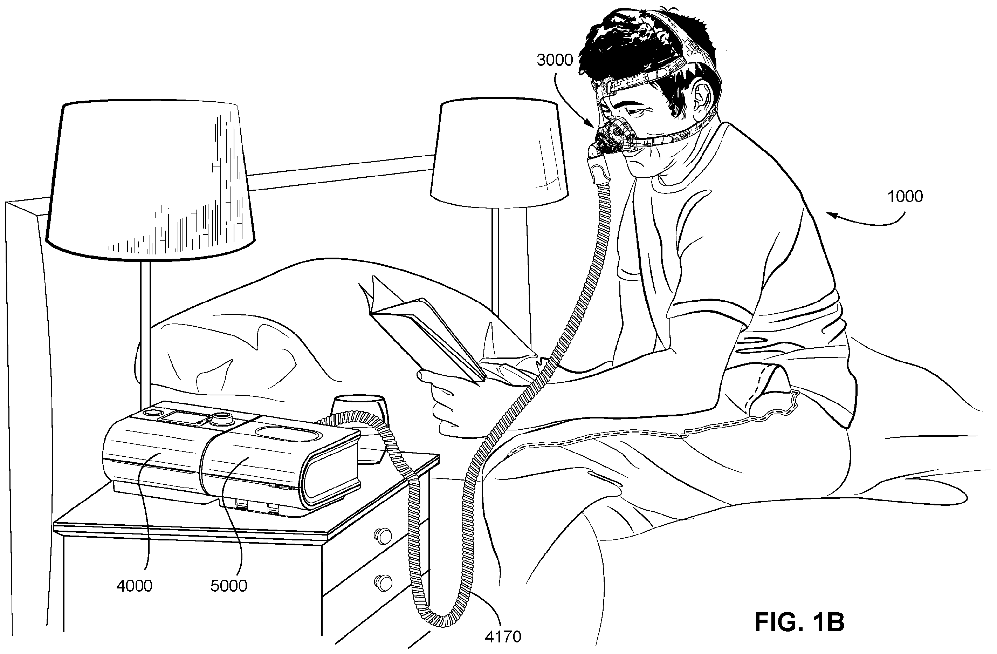

Fig. 1B shows a system including a patient 1000 wearing a patient interface 3000, in the form of a nasal mask, receiving a supply of air at positive pressure from an RPT device 4000. Air from the RPT device is humidified in a humidifier 5000, and passes along an air circuit 4170 to the patient 1000.

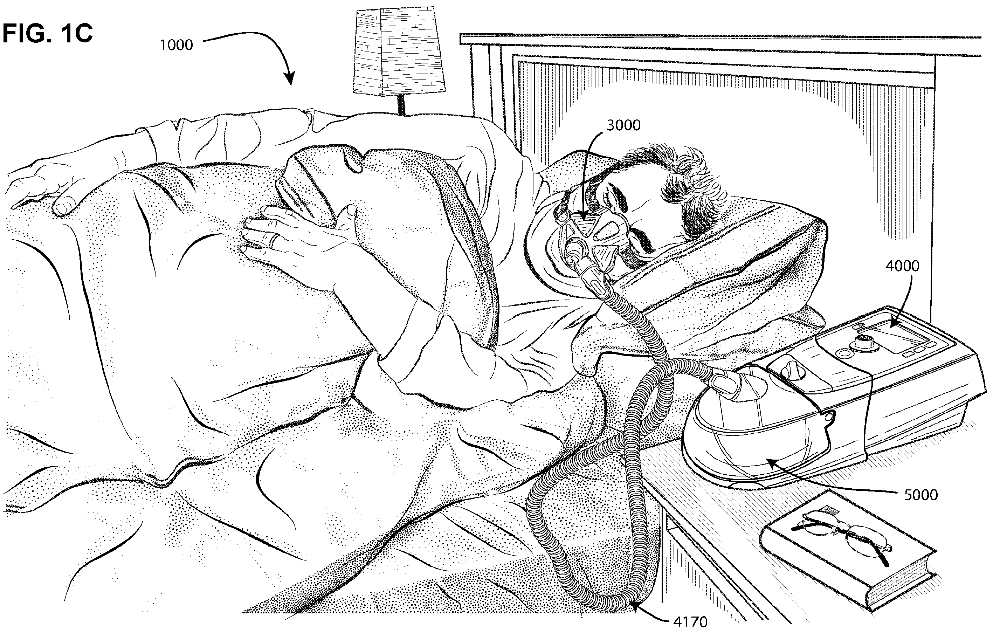

Fig. 1C shows a system including a patient 1000 wearing a patient interface 3000, in the form of a full-face mask, receiving a supply of air at positive pressure from an RPT device 4000. Air from the RPT device is humidified in a humidifier 5000, and passes along an air circuit 4170 to the patient 1000. The patient is sleeping in a side sleeping position.

### 3.2 RESPIRATORY SYSTEM AND FACIAL ANATOMY

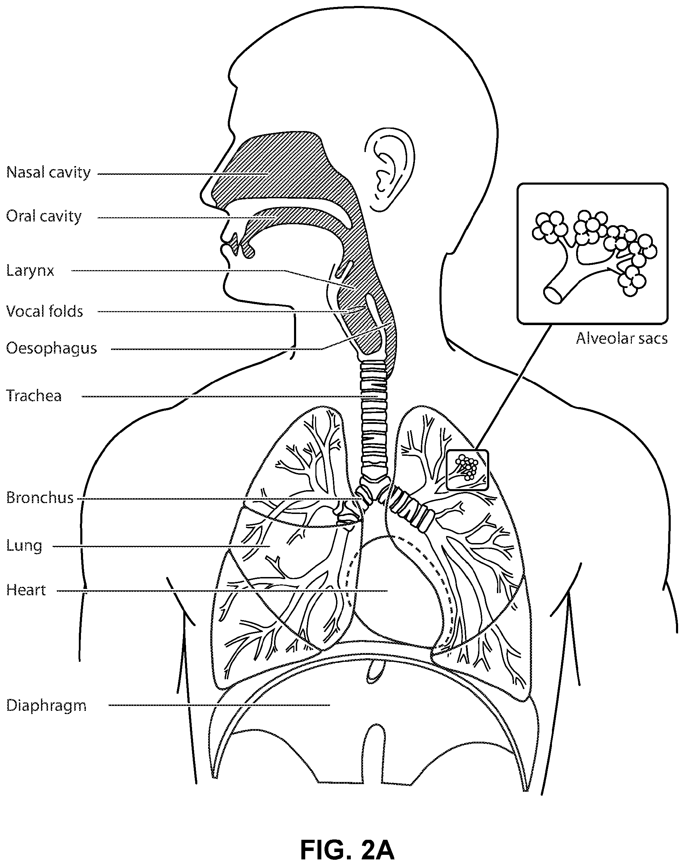

Fig. 2A shows an overview of a human respiratory system including the nasal and oral cavities, the larynx, vocal folds, oesophagus, trachea, bronchus, lung, alveolar sacs, heart and diaphragm.

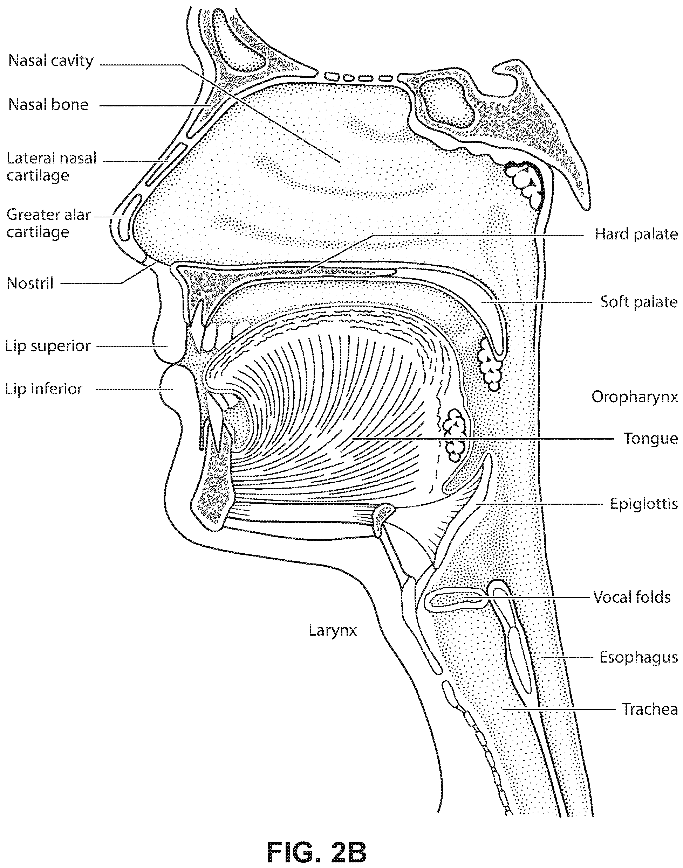

Fig. 2B shows a view of a human upper airway including the nasal cavity, nasal bone, lateral nasal cartilage, greater alar cartilage, nostril, lip superior, lip inferior, larynx, hard palate, soft palate, oropharynx, tongue, epiglottis, vocal folds, oesophagus and trachea.

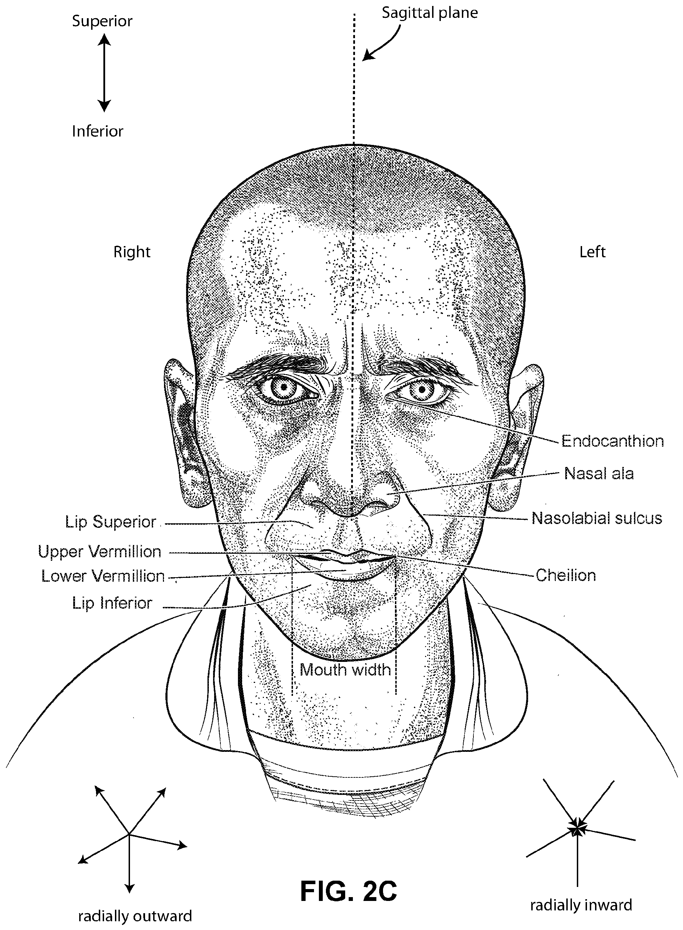

Fig. 2C is a front view of a face with several features of surface anatomy identified including the lip superior, upper vermilion, lower vermilion, lip inferior, mouth width, endocanthion, a nasal ala, nasolabial sulcus and cheilion. Also indicated are the directions superior, inferior, radially inward and radially outward.

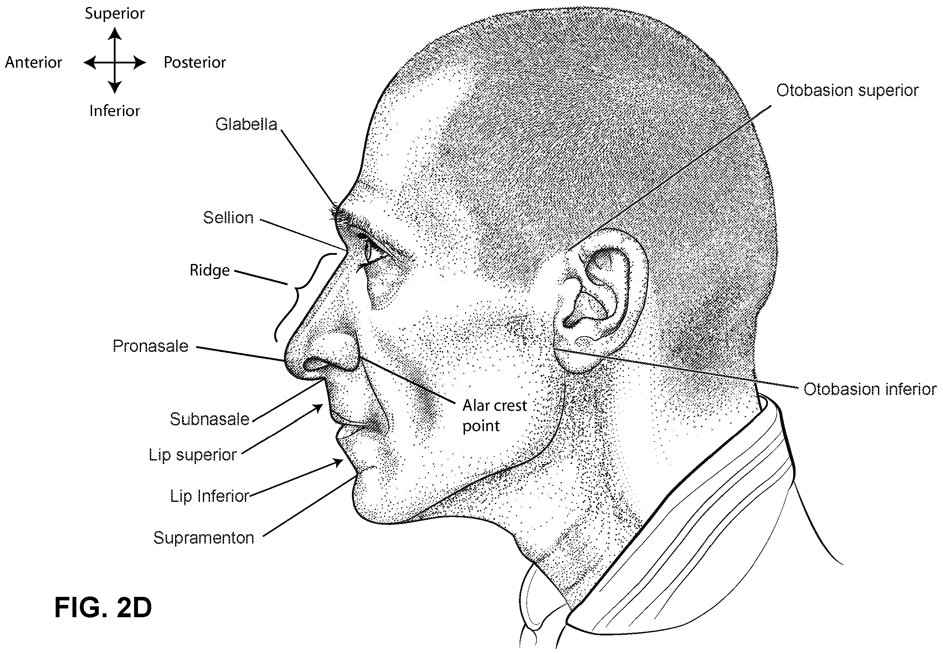

Fig. 2D is a side view of a head with several features of surface anatomy identified including glabella, sellion, pronasale, subnasale, lip superior, lip inferior, supramenton, nasal ridge, alar crest point, otobasion superior and otobasion inferior. Also indicated are the directions superior & inferior, and anterior & posterior.

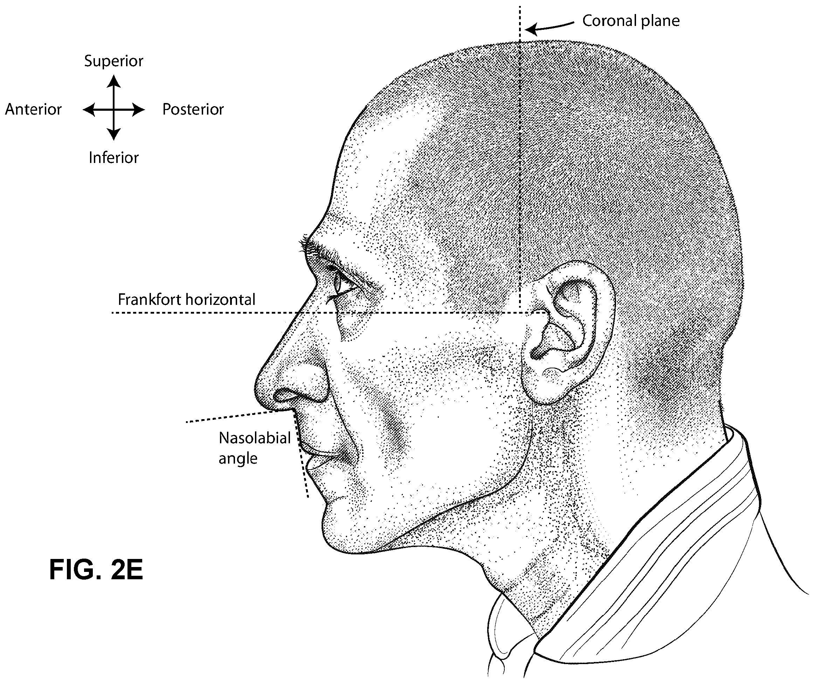

Fig. 2E is a further side view of a head. The approximate locations of the Frankfort horizontal and nasolabial angle are indicated. The coronal plane is also indicated.

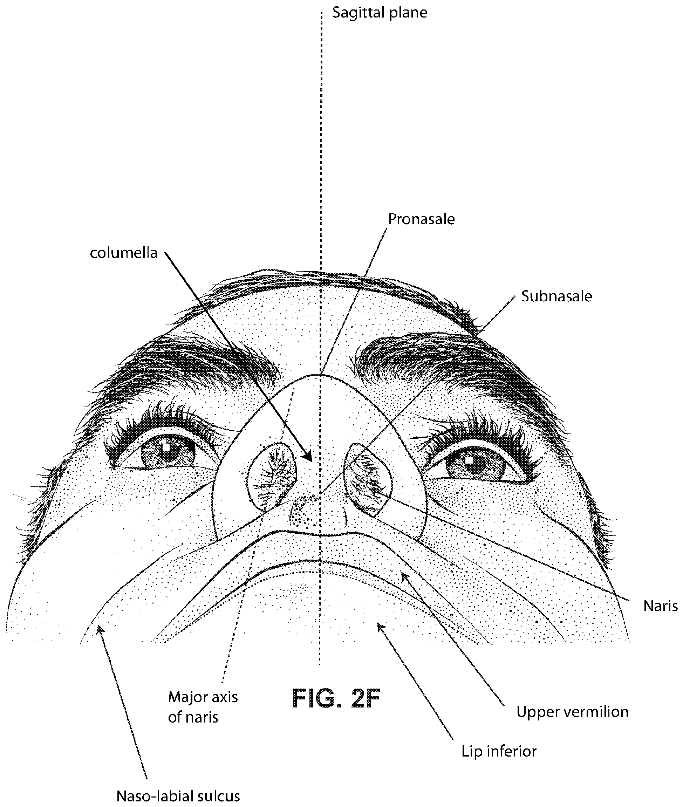

Fig. 2F shows a base view of a nose with several features identified including naso-labial sulcus, lip inferior, upper Vermilion, naris, subnasale, columella, pronasale, the major axis of a naris and the midsagittal plane.

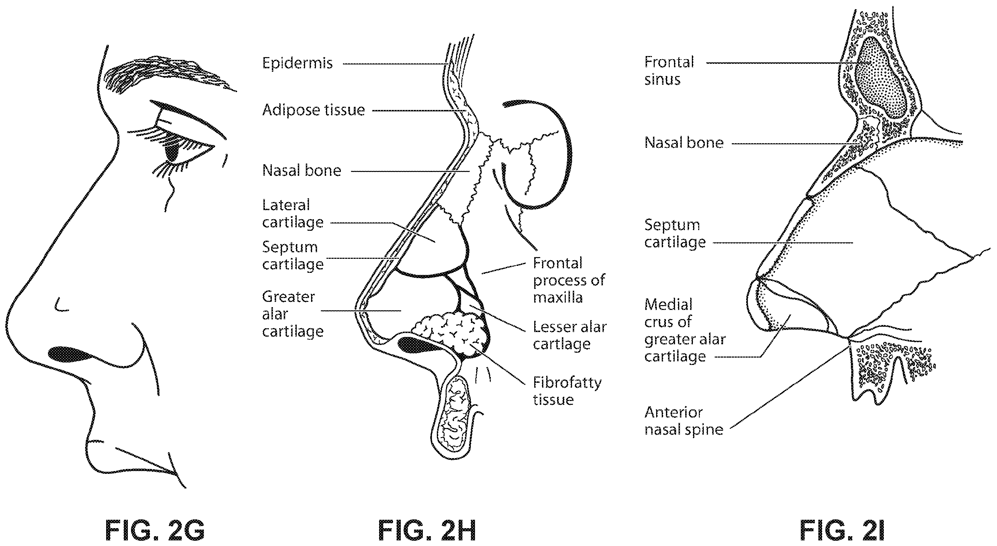

Fig. 2G shows a side view of the superficial features of a nose.
Fig. 2H shows subcutaneal structures of the nose, including lateral cartilage, septum cartilage, greater alar cartilage, lesser alar cartilage, sesamoid cartilage, nasal bone, epidermis, adipose tissue, frontal process of the maxilla and fibrofatty tissue.
Fig. 2I shows a medial dissection of a nose, approximately several millimeters from the midsagittal plane, amongst other things showing the septum cartilage and medial crus of greater alar cartilage.

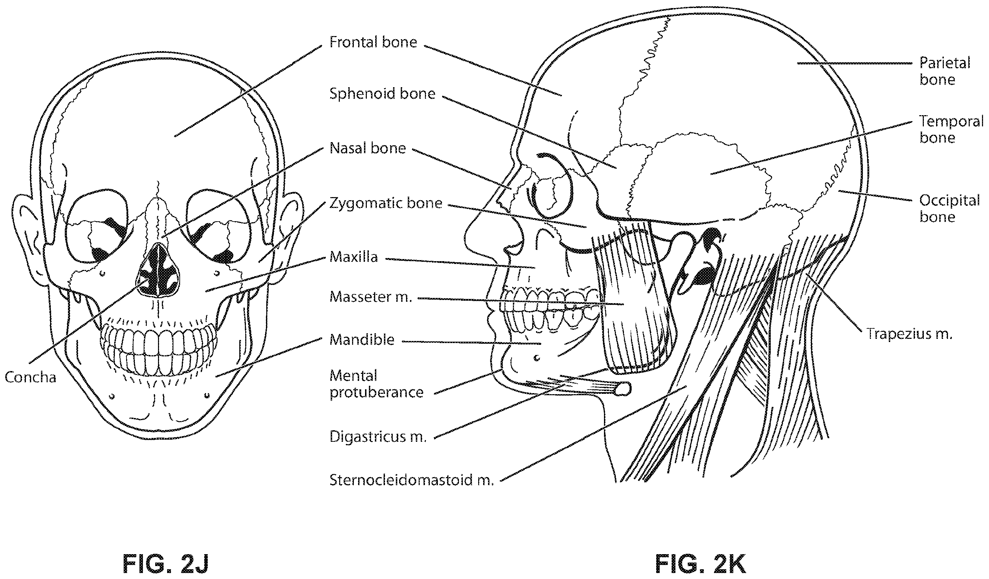

Fig. 2J shows a front view of the bones of a skull including the frontal, nasal and zygomatic bones. Nasal concha are indicated, as are the maxilla, and mandible.
Fig. 2K shows a lateral view of a skull with the outline of the surface of a head, as well as several muscles. The following bones are shown: frontal, sphenoid, nasal, zygomatic, maxilla, mandible, parietal, temporal and occipital. The mental protuberance is indicated. The following muscles are shown: digastricus, masseter, sternocleidomastoid and trapezius.

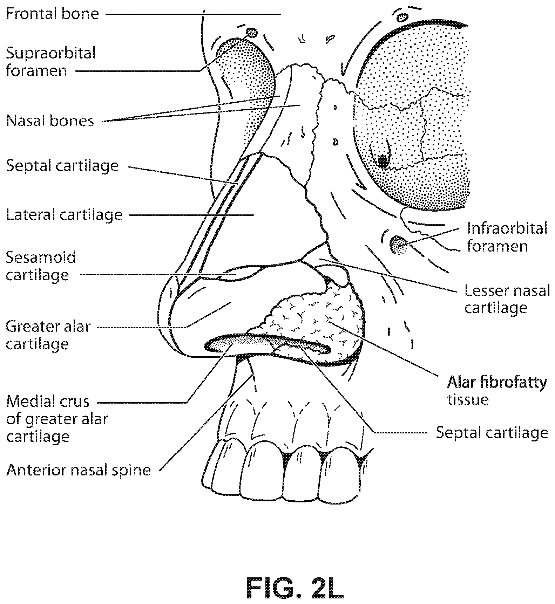

Fig. 2L shows an anterolateral view of a nose.

### 3.3 PATIENT INTERFACE

Fig. 3A shows a patient interface in the form of a nasal mask in accordance with one form of the present technology.
Fig. 3B shows a schematic of a cross-section through a structure at a point. An outward normal at the point is indicated. The curvature at the point has a positive sign, and a relatively large magnitude when compared to the magnitude of the curvature shown in Fig. 3C.
Fig. 3C shows a schematic of a cross-section through a structure at a point. An outward normal at the point is indicated. The curvature at the point has a positive sign, and a relatively small magnitude when compared to the magnitude of the curvature shown in Fig. 3B.
Fig. 3D shows a schematic of a cross-section through a structure at a point. An outward normal at the point is indicated. The curvature at the point has a value of zero.
Fig. 3E shows a schematic of a cross-section through a structure at a point. An outward normal at the point is indicated. The curvature at the point has a negative sign, and a relatively small magnitude when compared to the magnitude of the curvature shown in Fig. 3F.
Fig. 3F shows a schematic of a cross-section through a structure at a point. An outward normal at the point is indicated. The curvature at the point has a negative sign, and a relatively large magnitude when compared to the magnitude of the curvature shown in Fig. 3E.
Fig. 3G shows a cushion for a mask that includes two pillows. An exterior surface of the cushion is indicated. An edge of the surface is indicated. Dome and saddle regions are indicated.
Fig. 3H shows a cushion for a mask. An exterior surface of the cushion is indicated. An edge of the surface is indicated. A path on the surface between points A and B is indicated. A straight line distance between A and B is indicated. Two saddle regions and a dome region are indicated.
Fig. 3I shows the surface of a structure, with a one dimensional hole in the surface. The illustrated plane curve forms the boundary of a one dimensional hole.
Fig. 3J shows a cross-section through the structure of Fig.3I. The illustrated surface bounds a two dimensional hole in the structure of Fig. 3I.
Fig. 3K shows a perspective view of the structure of Fig. 3I, including the two dimensional hole and the one dimensional hole. Also shown is the surface that bounds a two dimensional hole in the structure of Fig. 3I.
Fig. 3L shows a mask having an inflatable bladder as a cushion.
Fig. 3M shows a cross-section through the mask of Fig. 3L, and shows the interior surface of the bladder. The interior surface bounds the two dimensional hole in the mask.
Fig. 3N shows a further cross-section through the mask of Fig. 3L. The interior surface is also indicated.
Fig. 3O illustrates a left-hand rule.
Fig. 3P illustrates a right-hand rule.
Fig. 3Q shows a left ear, including the left ear helix.
Fig. 3R shows a right ear, including the right ear helix.
Fig. 3S shows a right-hand helix.
Fig. 3T shows a view of a mask, including the sign of the torsion of the space curve defined by the edge of the sealing membrane in different regions of the mask.
Fig. 3U shows a view of a plenum chamber 3200 showing a sagittal plane and a mid-contact plane.
Fig. 3V shows a view of a posterior of the plenum chamber of Fig. 3U. The direction of the view is normal to the mid-contact plane. The sagittal plane in Fig. 3V bisects the plenum chamber into left-hand and right-hand sides.

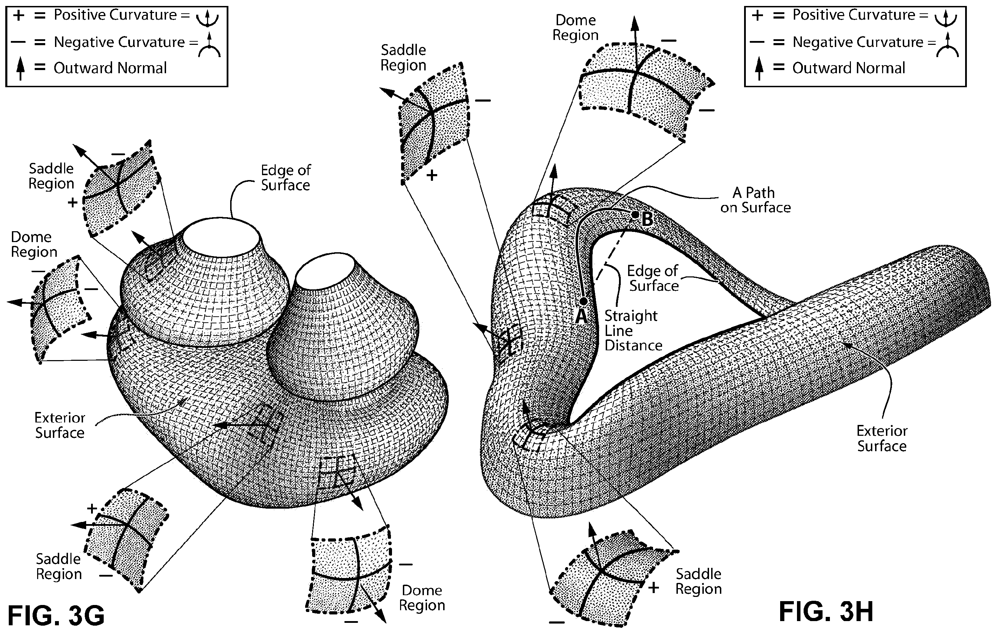

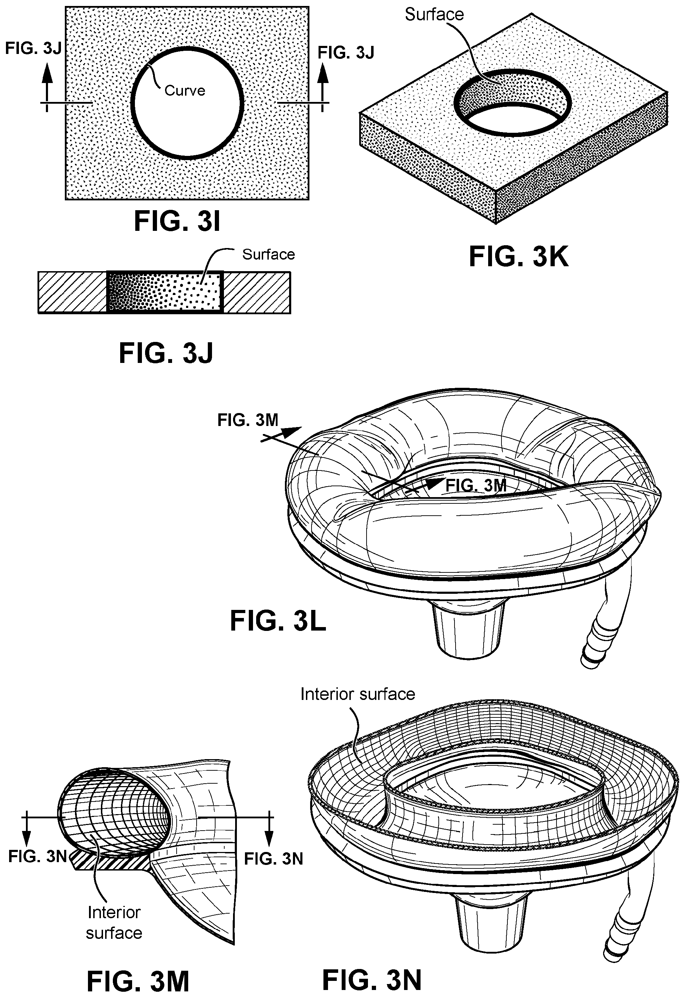

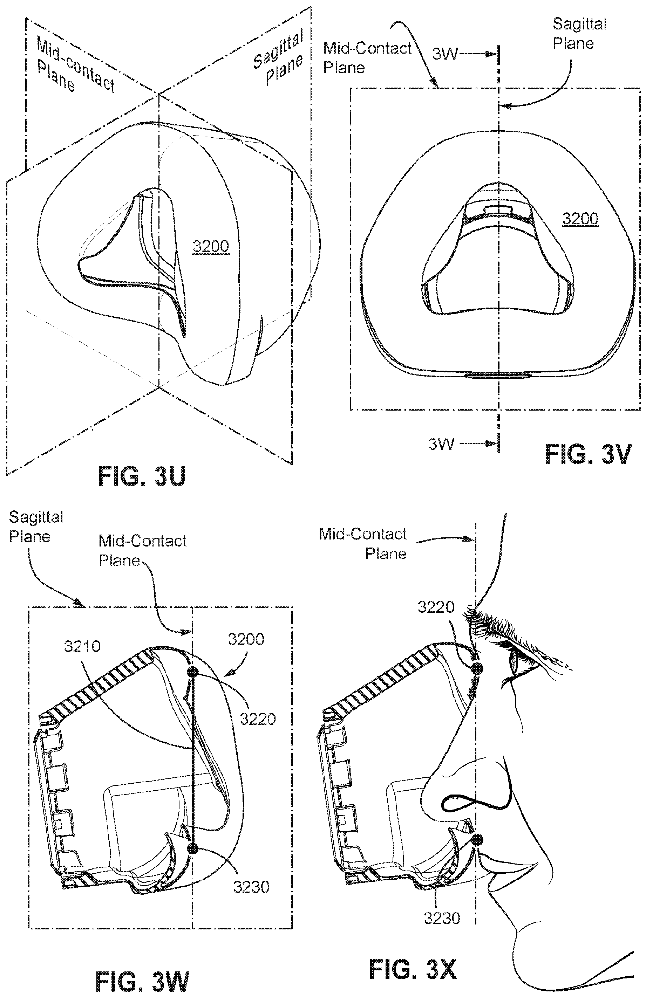

Fig. 3W shows a cross-section through the plenum chamber of Fig. 3V, the cross-section being taken at the sagittal plane shown in Fig. 3V. A 'mid-contact' plane is shown. The mid-contact plane is perpendicular to the sagittal plane. The orientation of the mid-contact plane corresponds to the orientation of a chord 3210 which lies on the sagittal plane and just touches the cushion of the plenum chamber at two points on the sagittal plane: a superior point 3220 and an inferior point 3230. Depending on the geometry of the cushion in this region, the mid-contact plane may be a tangent at both the superior and inferior points.
Fig. 3X shows the plenum chamber 3200 of Fig. 3U in position for use on a face. The sagittal plane of the plenum chamber 3200 generally coincides with the midsagittal plane of the face when the plenum chamber is in position for use. The mid-contact plane corresponds generally to the 'plane of the face' when the plenum chamber is in position for use. In Fig. 3X the plenum chamber 3200 is that of a nasal mask, and the superior point 3220 sits approximately on the sellion, while the inferior point 3230 sits on the lip superior.

### 3.4 RPT DEVICE

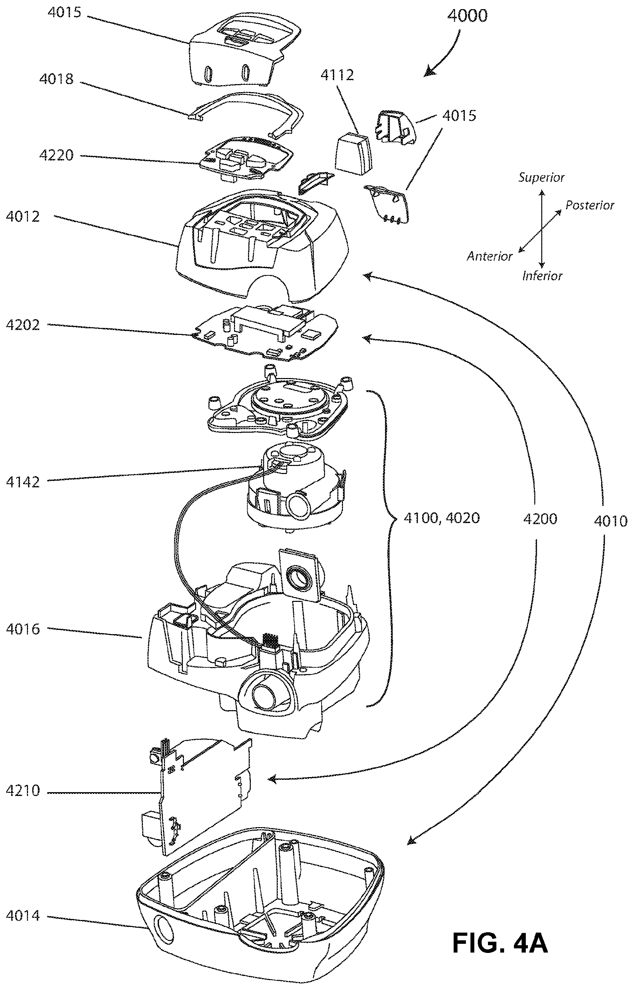

Fig. 4A shows an RPT device in accordance with one form of the present technology.

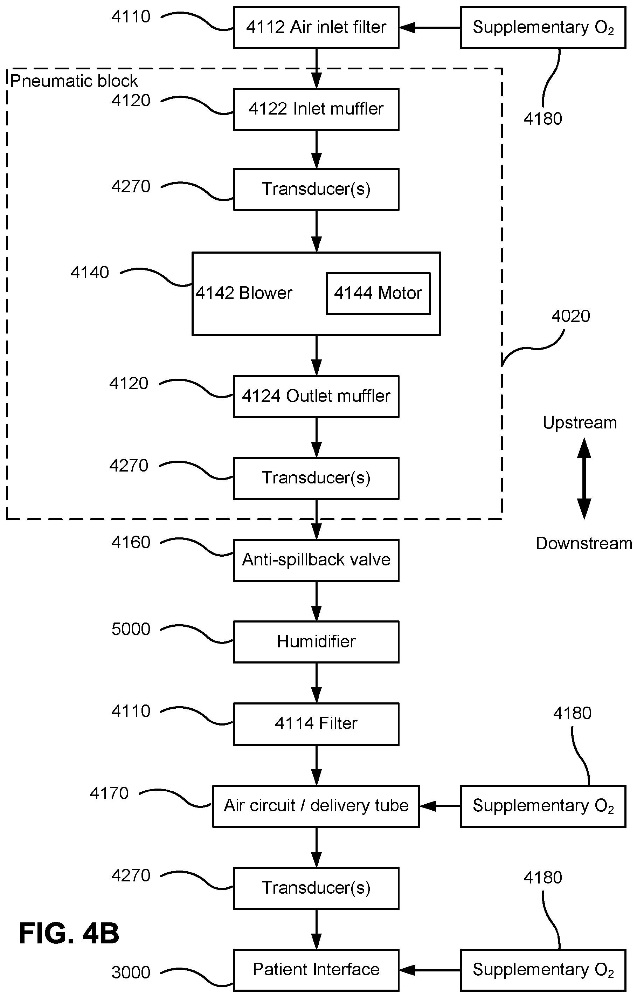

Fig. 4B is a schematic diagram of the pneumatic path of an RPT device in accordance with one form of the present technology. The directions of upstream and downstream are indicated with reference to the blower and the patient interface. The blower is defined to be upstream of the patient interface and the patient interface is defined to be downstream of the blower, regardless of the actual flow direction at any particular moment. Items which are located within the pneumatic path between the blower and the patient interface are downstream of the blower and upstream of the patient interface.

### 3.5 HUMIDIFIER

Fig. 5A shows an isometric view of a humidifier in accordance with one form of the present technology.
Fig. 5B shows an isometric view of a humidifier in accordance with one form of the present technology, showing a humidifier reservoir 5110 removed from the humidifier reservoir dock 5130.

### 3.6 BREATHING WAVEFORMS

Fig. 6 shows a model typical breath waveform of a person while sleeping.

### 3.7 CONNECTORS OF THE PRESENT TECHNOLOGY

Fig. 7 is a perspective view of a fluid connector of one form of the present technology in use connecting an air circuit to a patient interface, with the headgear and headgear connectors of the patient interface not shown.
Fig. 8 is a perspective view of a first connector part of the connector of Fig. 7.
Fig. 8A is a cross-section view of the first connector part of the connector shown in Fig. 7.
Fig. 9 is a perspective view of a second connector part of the connector of Fig. 7 engaged with an air circuit.
Fig. 10 shows a cross-section through the connector of Fig. 7, with the connector in a connected configuration and engaged with an air circuit.
Fig. 11 shows a cross-section through plane A-A.
Fig. 12 shows a cross-section through the connector of Fig. 7, with the first and second connector parts held such that each radially projecting portion engages a respective groove entrance, and with the connector engaged with an air circuit.
Fig. 13 shows a cross-section through the connector of Fig. 7 with the second connector part partially disconnected from the first connector part.
Fig. 14 shows a side view of a fluid connector of one form of the present technology, with the first and second connector parts in a fully connected configuration.
Fig. 15 shows a cross-section side view of the connector of Fig. 14.
Fig. 16 shows a perspective view of the connector of Fig. 14, with an upper half of the second connector part removed for clarity.
Fig. 17 shows a perspective view of the first connector part of the fluid connector of Fig. 14, with the magnetic portion removed.
Fig. 18 shows a view from one side of the first connector part of the fluid connector of Fig. 14.
Fig. 19 shows a view from the opposite side of the first connector part of the fluid connector of Fig. 14.
Fig. 20 shows a perspective view of the second connector part of connector of Fig. 14 with the magnetic portion removed.
Fig. 21 shows a view of the second connector part of the connector of Fig. 14 from the opposite end to the engaging face.
Fig. 22 shows a view of the second connector part of the connector of Fig. 14 from the engaging face end.
Fig. 23 shows a cross-section through plane B-B.
Fig. 24 shows a cross-section through plane C-C.
Fig. 25 shows a cross-section side view of the connector of Fig. 14 in a partially engaged configuration.

### 4 DETAILED DESCRIPTION OF EXAMPLES OF THE TECHNOLOGY

Before the present technology is described in further detail, it is to be understood that the technology is not limited to the particular examples described herein, which may vary. It is also to be understood that the terminology used in this disclosure is for the purpose of describing only the particular examples discussed herein, and is not intended to be limiting.

The following description is provided in relation to various examples which may share one or more common characteristics and/or features. It is to be understood that one or more features of any one example may be combinable with one or more features of another example or other examples. In addition, any single feature or combination of features in any of the examples may constitute a further example.

### 4.1 THERAPY

In one form, the present technology comprises a method for treating a respiratory disorder comprising applying positive pressure to the entrance of the airways of a patient 1000.

In certain examples of the present technology, a supply of air at positive pressure is provided to the nasal passages of the patient via one or both nares.

In certain examples of the present technology, mouth breathing is limited, restricted or prevented.

### 4.2 RESPIRATORY THERAPY SYSTEMS

In one form, the present technology comprises a respiratory therapy system for treating a respiratory disorder. The respiratory therapy system may comprise an RPT device 4000 for supplying a flow of air to the patient 1000 via an air circuit 4170 and a patient interface 3000.

### 4.3 PATIENT INTERFACE

A non-invasive patient interface 3000 in accordance with one aspect of the present technology comprises the following functional aspects: a seal-forming structure 3100, a plenum chamber 3200, a positioning and stabilising structure 3300, a vent 3400, one form of connection port 3600 for connection to air circuit 4170, and a forehead support 3700. In some forms a functional aspect may be provided by one or more physical components. In some forms, one physical component may provide one or more functional aspects. In use the seal-forming structure 3100 is arranged to surround an entrance to the airways of the patient so as to maintain positive pressure at the entrance(s) to the airways of the patient 1000. The sealed patient interface 3000 is therefore suitable for delivery of positive pressure therapy.

If a patient interface is unable to comfortably deliver a minimum level of positive pressure to the airways, the patient interface may be unsuitable for respiratory pressure therapy.

The patient interface 3000 in accordance with one form of the present technology is constructed and arranged to be able to provide a supply of air at a positive pressure of at least 6 cmH2O with respect to ambient.

The patient interface 3000 in accordance with one form of the present technology is constructed and arranged to be able to provide a supply of air at a positive pressure of at least 10 cmH2O with respect to ambient.

The patient interface 3000 in accordance with one form of the present technology is constructed and arranged to be able to provide a supply of air at a positive pressure of at least 20 cmH2O with respect to ambient.

### 4.3.1 Seal-forming structure

In one form of the present technology, a seal-forming structure 3100 provides a target seal-forming region, and may additionally provide a cushioning function. The target seal-forming region is a region on the seal-forming structure 3100 where sealing may occur. The region where sealing actually occurs- the actual sealing surface- may change within a given treatment session, from day to day, and from patient to patient, depending on a range of factors including for example, where the patient interface was placed on the face, tension in the positioning and stabilising structure and the shape of a patient's face.

In one form the target seal-forming region is located on an outside surface of the seal-forming structure 3100.

In certain forms of the present technology, the seal-forming structure 3100 is constructed from a biocompatible material, e.g. silicone rubber.

A seal-forming structure 3100 in accordance with the present technology may be constructed from a soft, flexible, resilient material such as silicone.

In certain forms of the present technology, a system is provided comprising more than one a seal-forming structure 3100, each being configured to correspond to a different size and/or shape range. For example the system may comprise one form of a seal-forming structure 3100 suitable for a large sized head, but not a small sized head and another suitable for a small sized head, but not a large sized head.

### 4.3.1.1 Sealing mechanisms

In one form, the seal-forming structure includes a sealing flange utilizing a pressure assisted sealing mechanism. In use, the sealing flange can readily respond to a system positive pressure in the interior of the plenum chamber 3200 acting on its underside to urge it into tight sealing engagement with the face. The pressure assisted mechanism may act in conjunction with elastic tension in the positioning and stabilising structure.

In one form, the seal-forming structure 3100 comprises a sealing flange and a support flange. The sealing flange comprises a relatively thin member with a thickness of less than about 1mm, for example about 0.25mm to about 0.45mm, which extends around the perimeter of the plenum chamber 3200. Support flange may be relatively thicker than the sealing flange. The support flange is disposed between the sealing flange and the marginal edge of the plenum chamber 3200, and extends at least part of the way around the perimeter. The support flange is or includes a springlike element and functions to support the sealing flange from buckling in use.

In one form, the seal-forming structure may comprise a compression sealing portion or a gasket sealing portion. In use the compression sealing portion, or the gasket sealing portion is constructed and arranged to be in compression, e.g. as a result of elastic tension in the positioning and stabilising structure.

In one form, the seal-forming structure comprises a tension portion. In use, the tension portion is held in tension, e.g. by adjacent regions of the sealing flange.

In one form, the seal-forming structure comprises a region having a tacky or adhesive surface.

In certain forms of the present technology, a seal-forming structure may comprise one or more of a pressure-assisted sealing flange, a compression sealing portion, a gasket sealing portion, a tension portion, and a portion having a tacky or adhesive surface.

### 4.3.1.2 Nose bridge or nose ridge region

In one form, the non-invasive patient interface 3000 comprises a seal-forming structure that forms a seal in use on a nose bridge region or on a nose-ridge region of the patient's face.

In one form, the seal-forming structure includes a saddle-shaped region constructed to form a seal in use on a nose bridge region or on a nose-ridge region of the patient's face.

### 4.3.1.3 Upper lip region

In one form, the non-invasive patient interface 3000 comprises a seal-forming structure that forms a seal in use on an upper lip region (that is, the lip superior) of the patient's face.

In one form, the seal-forming structure includes a saddle-shaped region constructed to form a seal in use on an upper lip region of the patient's face.

### 4.3.1.4 Chin-region

In one form the non-invasive patient interface 3000 comprises a seal-forming structure that forms a seal in use on a chin-region of the patient's face.

In one form, the seal-forming structure includes a saddle-shaped region constructed to form a seal in use on a chin-region of the patient's face.

### 4.3.1.5 Forehead region

In one form, the seal-forming structure that forms a seal in use on a forehead region of the patient's face. In such a form, the plenum chamber may cover the eyes in use.

### 4.3.1.6 Nasal pillows

In one form the seal-forming structure of the non-invasive patient interface 3000 comprises a pair of nasal puffs, or nasal pillows, each nasal puff or nasal pillow being constructed and arranged to form a seal with a respective naris of the nose of a patient.

Nasal pillows in accordance with an aspect of the present technology include: a frusto-cone, at least a portion of which forms a seal on an underside of the patient's nose, a stalk, a flexible region on the underside of the frusto-cone and connecting the frusto-cone to the stalk. In addition, the structure to which the nasal pillow of the present technology is connected includes a flexible region adjacent the base of the stalk. The flexible regions can act in concert to facilitate a universal joint structure that is accommodating of relative movement both displacement and angular of the frusto-cone and the structure to which the nasal pillow is connected. For example, the frusto-cone may be axially displaced towards the structure to which the stalk is connected.

### 4.3.2 Plenum chamber

The plenum chamber 3200 has a perimeter that is shaped to be complementary to the surface contour of the face of an average person in the region where a seal will form in use. In use, a marginal edge of the plenum chamber 3200 is positioned in close proximity to an adjacent surface of the face. Actual contact with the face is provided by the seal-forming structure 3100. The seal-forming structure 3100 may extend in use about the entire perimeter of the plenum chamber 3200. In some forms, the plenum chamber 3200 and the seal-forming structure 3100 are formed from a single homogeneous piece of material.

In certain forms of the present technology, the plenum chamber 3200 does not cover the eyes of the patient in use. In other words, the eyes are outside the pressurised volume defined by the plenum chamber. Such forms tend to be less obtrusive and / or more comfortable for the wearer, which can improve compliance with therapy.

In certain forms of the present technology, the plenum chamber 3200 is constructed from a transparent material, e.g. a transparent polycarbonate. The use of a transparent material can reduce the obtrusiveness of the patient interface, and help improve compliance with therapy. The use of a transparent material can aid a clinician to observe how the patient interface is located and functioning.

In certain forms of the present technology, the plenum chamber 3200 is constructed from a translucent material. The use of a translucent material can reduce the obtrusiveness of the patient interface, and help improve compliance with therapy.

### 4.3.3 Positioning and stabilising structure

The seal-forming structure 3100 of the patient interface 3000 of the present technology may be held in sealing position in use by the positioning and stabilising structure 3300.

In one form the positioning and stabilising structure 3300 provides a retention force at least sufficient to overcome the effect of the positive pressure in the plenum chamber 3200 to lift off the face.

In one form the positioning and stabilising structure 3300 provides a retention force to overcome the effect of the gravitational force on the patient interface 3000.

In one form the positioning and stabilising structure 3300 provides a retention force as a safety margin to overcome the potential effect of disrupting forces on the patient interface 3000, such as from tube drag, or accidental interference with the patient interface.

In one form of the present technology, a positioning and stabilising structure 3300 is provided that is configured in a manner consistent with being worn by a patient while sleeping. In one example the positioning and stabilising structure 3300 has a low profile, or cross-sectional thickness, to reduce the perceived or actual bulk of the apparatus. In one example, the positioning and stabilising structure 3300 comprises at least one strap having a rectangular cross-section. In one example the positioning and stabilising structure 3300 comprises at least one flat strap.

In one form of the present technology, a positioning and stabilising structure 3300 is provided that is configured so as not to be too large and bulky to prevent the patient from lying in a supine sleeping position with a back region of the patient's head on a pillow.

In one form of the present technology, a positioning and stabilising structure 3300 is provided that is configured so as not to be too large and bulky to prevent the patient from lying in a side sleeping position with a side region of the patient's head on a pillow.

In one form of the present technology, a positioning and stabilising structure 3300 is provided with a decoupling portion located between an anterior portion of the positioning and stabilising structure 3300, and a posterior portion of the positioning and stabilising structure 3300. The decoupling portion does not resist compression and may be, e.g. a flexible or floppy strap. The decoupling portion is constructed and arranged so that when the patient lies with their head on a pillow, the presence of the decoupling portion prevents a force on the posterior portion from being transmitted along the positioning and stabilising structure 3300 and disrupting the seal.

In one form of the present technology, a positioning and stabilising structure 3300 comprises a strap constructed from a laminate of a fabric patient-contacting layer, a foam inner layer and a fabric outer layer. In one form, the foam is porous to allow moisture, (e.g., sweat), to pass through the strap. In one form, the fabric outer layer comprises loop material to engage with a hook material portion.

In certain forms of the present technology, a positioning and stabilising structure 3300 comprises a strap that is extensible, e.g. resiliently extensible. For example the strap may be configured in use to be in tension, and to direct a force to draw a seal-forming structure into sealing contact with a portion of a patient's face. In an example the strap may be configured as a tie.

In one form of the present technology, the positioning and stabilising structure comprises a first tie, the first tie being constructed and arranged so that in use at least a portion of an inferior edge thereof passes superior to an otobasion superior of the patient's head and overlays a portion of a parietal bone without overlaying the occipital bone.

In one form of the present technology suitable for a nasal-only mask or for a full-face mask, the positioning and stabilising structure includes a second tie, the second tie being constructed and arranged so that in use at least a portion of a superior edge thereof passes inferior to an otobasion inferior of the patient's head and overlays or lies inferior to the occipital bone of the patient's head.

In one form of the present technology suitable for a nasal-only mask or for a full-face mask, the positioning and stabilising structure includes a third tie that is constructed and arranged to interconnect the first tie and the second tie to reduce a tendency of the first tie and the second tie to move apart from one another.

In certain forms of the present technology, a positioning and stabilising structure 3300 comprises a strap that is bendable and e.g. non-rigid. An advantage of this aspect is that the strap is more comfortable for a patient to lie upon while the patient is sleeping.

In certain forms of the present technology, a positioning and stabilising structure 3300 comprises a strap constructed to be breathable to allow moisture vapour to be transmitted through the strap,

In certain forms of the present technology, a system is provided comprising more than one positioning and stabilizing structure 3300, each being configured to provide a retaining force to correspond to a different size and/or shape range. For example the system may comprise one form of positioning and stabilizing structure 3300 suitable for a large sized head, but not a small sized head, and another. suitable for a small sized head, but not a large sized head.

### 4.3.4 Vent

In one form, the patient interface 3000 includes a vent 3400 constructed and arranged to allow for the washout of exhaled gases, e.g. carbon dioxide.

In certain forms the vent 3400 is configured to allow a continuous vent flow from an interior of the plenum chamber 3200 to ambient whilst the pressure within the plenum chamber is positive with respect to ambient. The vent 3400 is configured such that the vent flow rate has a magnitude sufficient to reduce rebreathing of exhaled CO2 by the patient while maintaining the therapeutic pressure in the plenum chamber in use.

One form of vent 3400 in accordance with the present technology comprises a plurality of holes, for example, about 20 to about 80 holes, or about 40 to about 60 holes, or about 45 to about 55 holes.

The vent 3400 may be located in the plenum chamber 3200. Alternatively, the vent 3400 is located in a decoupling structure, e.g., a swivel.

### 4.3.5 Decoupling structure(s)

In one form the patient interface 3000 includes at least one decoupling structure, for example, a swivel or a ball and socket.

### 4.3.6 Connection port

Connection port 3600 allows for connection to the air circuit 4170.

### 4.3.7 Forehead support

In one form, the patient interface 3000 includes a forehead support 3700.

### 4.3.8 Anti-asphyxia valve

In one form, the patient interface 3000 includes an anti-asphyxia valve.

### 4.3.9 Ports

In one form of the present technology, a patient interface 3000 includes one or more ports that allow access to the volume within the plenum chamber 3200. In one form this allows a clinician to supply supplementary oxygen. In one form, this allows for the direct measurement of a property of gases within the plenum chamber 3200, such as the pressure.

### 4.3.10 Magnetic Fluid Connector

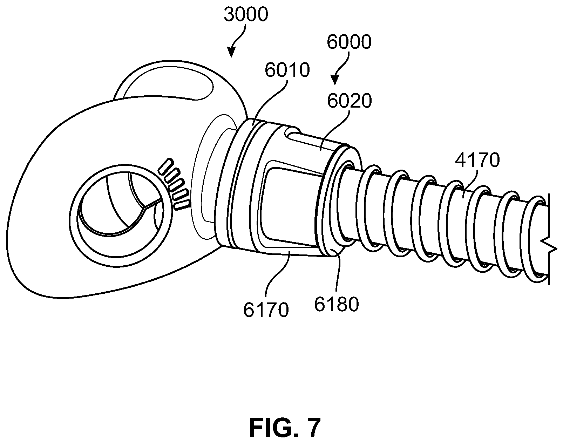

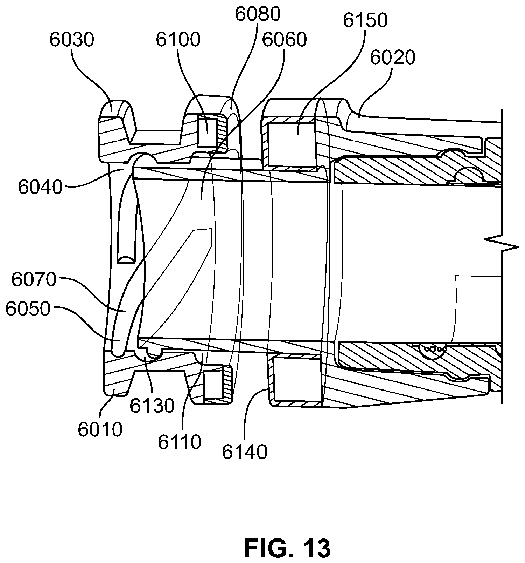

Referring next to Figs. 7 to 13, a fluid connector 6000 for coupling an air circuit 4170 to a respiratory therapy system component (e.g. a patient interface 3000, humidifier 5000 or RPT device 4000), to allow a flow of breathable gas, comprises a first connector part 6010 and a second connector part 6020.

The first connector part 6010 comprises a body 6030 having a passage 6032 which extends through the body 6030. The passage 6032 comprises a substantially tubular inner wall 6040.

The tubular inner wall 6040 is provided with at least one spiral channel or groove 6050 which extends from a first end 6052 of the tubular inner wall 6040. In examples, a plurality of spiral grooves 6050, for example six spiral grooves 6050, may be provided.

Each spiral groove 6050 has a groove entrance or opening 6060. In examples, each groove entrance 6060 is wider (e.g. in the circumferential direction) than the remainder of the groove. The spiral grooves 6050 and spiral groove entrances 6060 are circumferentially spaced apart, preferably evenly. Areas between the openings 6060 are referred to herein as lands 6160.

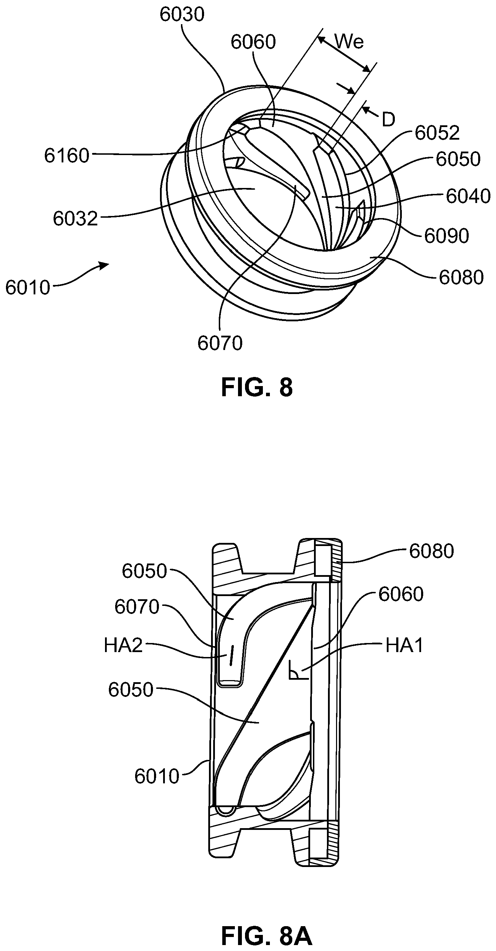

In some forms of the technology, the spiral grooves 6050 may have a substantially constant helix angle. However, in other forms of the technology the helix angle may change along the length of the groove. For example, as shown in Fig. 8A, in some examples the helix angle decreases toward the end of the groove 6050 distal the entrance 6060. In one form each spiral groove 6050 has a helix angle HA1 of substantially 90 degrees adjacent the groove entrance 6060, reducing to a helix angle HA2 of between 10 degrees and 0 degrees (e.g. substantially 1 degree) at the distal end 6070 of the groove.

A radially extending wall 6080 extends radially outwardly from the end 6090 of the passage 6032. In examples the radially extending wall 6080 has an annular shape. At least one magnetic portion and/or a ferromagnetic portion 6100 is provided behind the radially extending wall 6080, e.g. around 0.4 mm from the surface of the wall 6080. In one example the at least one magnetic portion and/or a ferromagnetic portion 6100 comprises an annular ferrite component.

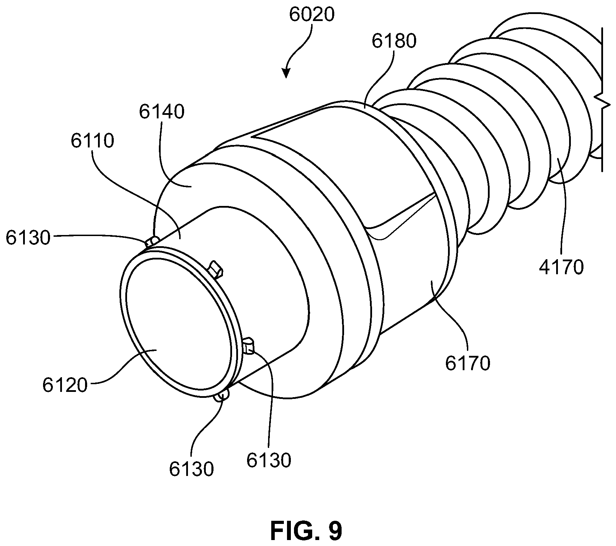

Referring next to Figs. 9, 11 and 12 in particular, the second connector part 6020 comprises a tubular outer wall 6110 which is configured to be received by the tubular inner wall 6040. In examples, the tubular outer wall 6110 is a sliding fit with the tubular inner wall 6040. The second connector part 6020 has a passage 6120 for air flow through the part 6020.

The tubular outer wall 6110 is provided with at least one radially projecting portion 6130. In examples, the number of radially projecting portions 6130 provided is equal to the number of spiral grooves 6050. The radially projecting portions 6130 may be evenly circumferentially spaced apart.

The second connector part 6020 comprises a radially extending wall 6140 extending radially outward from the tubular outer wall 6110. In examples the radially extending wall 6140 has an annular shape. In examples the radially projecting portions 6130 are provided to the end of the tubular outer wall 6110 distal the radially extending wall 6140.

At least one magnetic portion 6150 is provided behind the radially extending wall 6140, e.g. around 0.4 mm behind the surface of the wall. In one example the at least one magnetic portion 6150 comprises an annular magnetic component.

The first connector part 6010 and second connector part 6020 are engaged together by inserting the tubular outer wall 6110 into the tubular inner wall 6040. During this engagement each radially projecting portion 6130 enters a respective one of the spiral grooves 6050 and follows the groove. The motion of the projecting portions 6130 in the spiral grooves 6050 causes relative rotation of the first and second connector parts 6010, 6020 (or at least portions of those parts) as the two parts are moved toward each other.

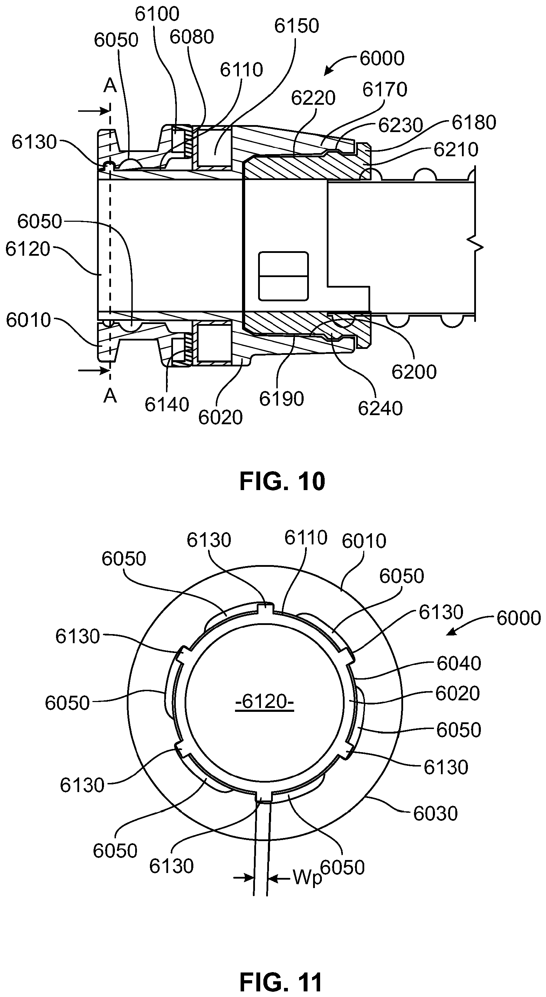

When the second connector part 6020 is fully engaged with the first connector part 6010, the radially extending walls 6080, 6140 abut each other, as shown in Figs. 7 and 10. When in this configuration, air flow between the first and second radially extending walls 6080, 6140 is blocked or eliminated. In one example one or both of the walls 6080, 6140 is provided with, or formed from, a resilient material (e.g. rubber or silicone) to improve the seal between the walls 6080, 6140. However, in other examples such resilient material may not be required, e.g. if the mating between the walls 6080, 6140 is sufficiently close that little or no air escapes. Alternative sealing means (e.g. O-ring seals) may also be used to prevent the escape of air from between the two connector parts 6010, 6020.

When in the connected configuration there is a magnetic attractive force between the magnetic portion 6150 and the magnetic portion and/or ferromagnetic portion 6100 which biases the first and second radially extending walls 6080, 6140 toward each other, that is, the force biases the connector parts 6010, 6020 to remain fully connected.

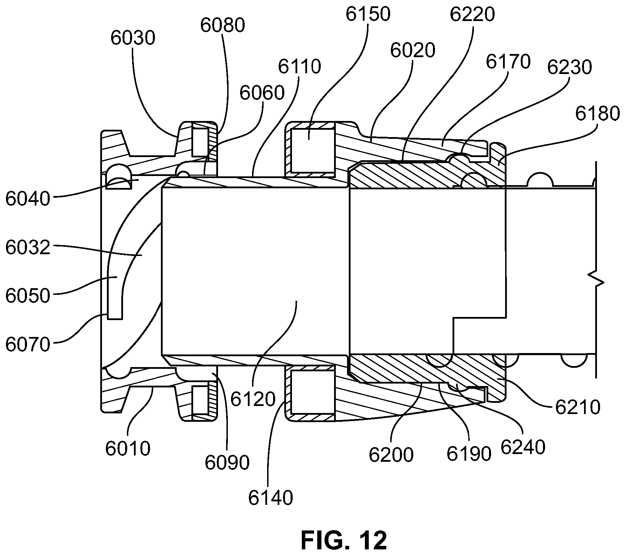

In examples of the disclosure, the magnetic attractive force between the magnetic portion 6150 and the magnetic portion and/or ferromagnetic portion 6100 is sufficient that when the second connector part 6020 is positioned relative to the first connector part 6010 such that the or each radially projecting portion 6130 engages a respective groove entrance 6060 (e.g. as seen in Fig. 12), the magnetic attractive force is sufficient to move the first and second connector parts to the connected configuration (e.g. as shown in Fig. 10) in the absence of any external force on the connector parts 6010, 6020. In this way, to engage the two connector parts 6010, 6020 the patient need only move the two parts 6010, 6020 into contact with each other, following which the connector 6000 may automatically move to a fully connected configuration. In some examples it may be necessary to move the two connector parts 6010, 6020 together such that the radially extending walls 6080, 6140 are between 3-5 mm apart before the connector 6000 automatically moves to the fully connected configuration. The distance at which the connector moves automatically may vary depending on the strength of the magnets.

In examples, the width We of each spiral groove entrance 6060 is significantly greater than the width Wp of each radially projecting portion. In examples, the distance D between adjacent spiral groove entrances 6060 is substantially ½ the width Wp of each radially projecting portion. These factors mean that there is an increased chance of the radially projecting portions 6130 aligning with the spiral groove entrances 6060 when the two parts 6010, 6020 are brought together.

When the distance D between each spiral groove entrance 6060 is small and/or the width Wp of each radially projecting portion 6130 is small, there is a reduced chance of the projecting portions 6130 aligning with the lands 6160 between the groove entrances 6060 rather than aligning with the groove entrances 6060 themselves. This may mean that typically the patient need not pay attention to deliberately aligning the radially projecting portions 6130 with the groove entrances 6060 when connecting the two parts 6010, 6020 together. In examples, the lands 6160 between the groove entrances 6060 may be angled, such that even if the radially projecting portions 6130 contact the lands 6160, one or both of the parts 6010, 6020 tend to rotate such that the radially projecting portions 6130 move toward the groove entrances 6060.

In one example the magnetic attractive force is substantially 2-4 N when the second connector part 6020 is positioned relative to the first connector part 6010 such that the or each radially projecting portion 6130 engages a respective groove entrance 6060, and is substantially 10-20N when the two parts 6010, 6020 are fully engaged. In examples the engagement of the radially projecting portions 6130 with the spiral grooves 6050 results in the axial force necessary to separate the first and second parts 6010, 6020 (when in the fully connected configuration) being greater than the magnetic attractive force, thereby increasing the security of the connection.

When in the fully connected configuration the first and second parts 6010, 6020 can conveniently be moved apart by rotating one of the parts 6010, 6020 (or at least a portion of the part) relative to the other. Such a rotation causes the radially projecting portions 6130 to follow the spiral groove 6050 and to thereby separate the two parts (e.g. to increase the separation between the magnetically attracting parts). Because the magnetic attractive force is substantially axial, and does not include a torque component, relative rotation of the first and second parts 6010, 6020 may be relatively easy, particularly in examples which have a reduced helix angle HA2 at the distal end 6070 of each groove 6050 (that being the operative part of the groove when the radially extending walls 6080, 6140 are abutting or closely spaced and the magnetic attractive force is at its greatest).

In one example the second connector part 6020 comprises a first body part 6170 and a second body part 6180, wherein the first body part 6170 is rotatable relative to the second body part 6180. In the example shown in Fig. 10, the first body part 6170 has a socket 6190 with an inner wall 6200 shaped as a surface of revolution, and the second part 6180 comprises a collar 6210 with a complementary outer surface 6220. In examples the socket 6190 has an annular channel 6230 which is engaged by an annular rib 6240 provided to the collar 6210, the engagement between the channel 6230 and rib 6240 keeping the collar 6210 and socket 6190 in engagement but allowing relative rotation.

In one example, one or both of the connector parts 6010, 6020 comprises a cuff for connection to a respiratory therapy system component. In one example the cuff may be a standard 22 mm (external diameter) ISO (International Organization for Standardization)-taper connector used in medical devices. In other examples the cuff may be structured for use with non-ISO standard connectors.

In examples the one of the connector parts 6010, 6020 may be permanently engaged with, or formed integrally with, a patient interface 3000, air circuit 4170, RPT 4000 or humidifier 5000.

In alternative examples the first connector part 6020 may be provided with a magnet and the second connector part 6020 may be provided with a ferrite component. In other examples both connector parts may be provided with magnets.

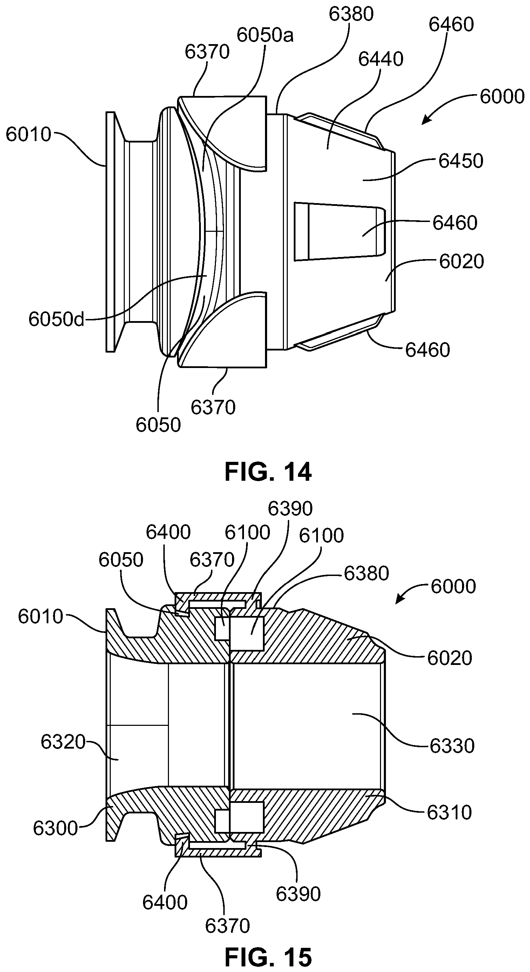

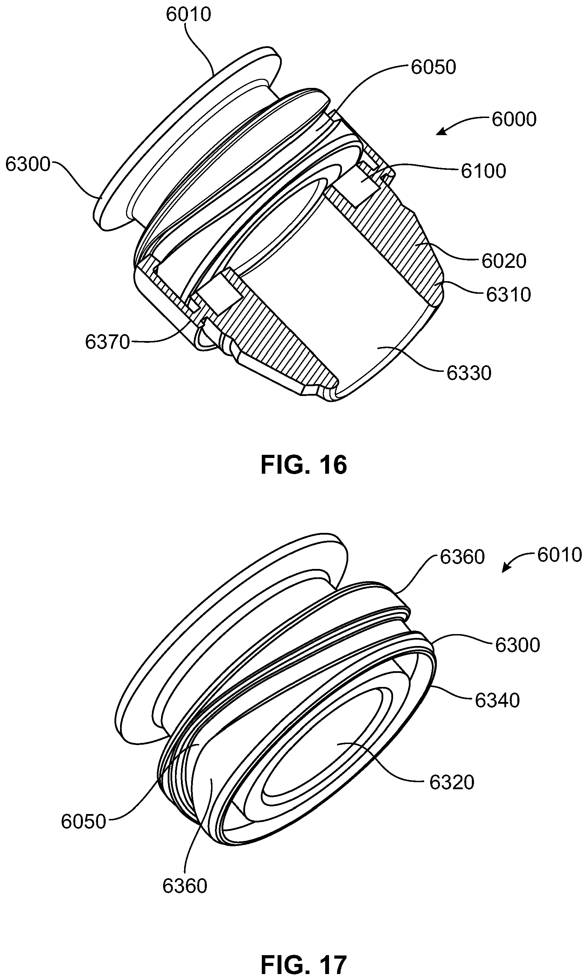

Referring next to Figs. 14 - 25, a fluid connector 6000 according to another form of the technology is shown.

The connector comprises a first connector part 6010 and a second connector part 6020. Each connector part 6010, 6020 comprises a body 6300, 6310 and a passage 6320, 6330 through the body 6300, 6310.

Each connector part body 6300, 6310 comprises a magnetic portion 6100 at or adjacent an engaging face 6340, 6350 thereof, or alternatively, one of the connector parts comprises a magnetic portion and the other comprises a ferromagnetic portion. The engaging faces 6340, 6350 define the first end of each body 6300, 6310 and are in contact when the connector is fully engaged. In examples, each connector part 6010, 6020 may comprise a plurality of magnetic portions or ferromagnetic portions 6100. In other examples, the magnetic and/or ferromagnetic parts may have an annular shape.

The first connector part body 6300 comprises a substantially cylindrical outer wall 6360. The cylindrical outer wall 6360 is provided with a channel or groove 6050. In examples, only a single groove 6050 is provided.

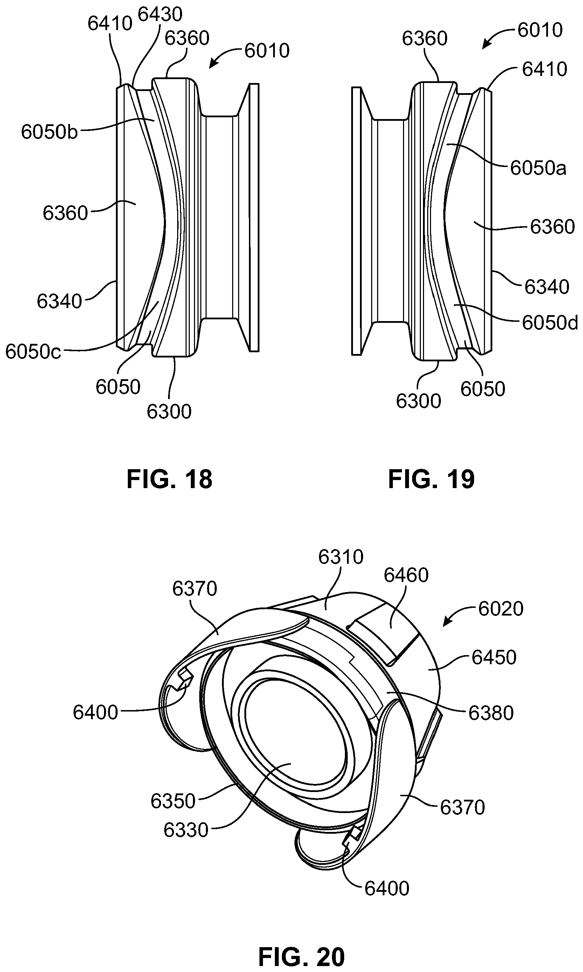

In the example shown, the groove 6050 comprises four contiguous substantially helical portions 6050a, 6050b, 6050c, 6050d having alternating orientations, as best seen in Figs. 18 and 19.

In the example shown, the groove 6050 is not provided with an entrance or opening. However, as is described further below, in examples an opening (e.g. an entrance/exit) is provided where the groove 6050 is closest to the first end of the body 6300.

The second connector part 6020 is provided with a plurality of longitudinally extending arms 6370 which extend in front of the engaging face 6350 of the body 6310, each of which is connected to an external wall 6380 of the second connector part body 6310 by a respective mounting portion 6390.

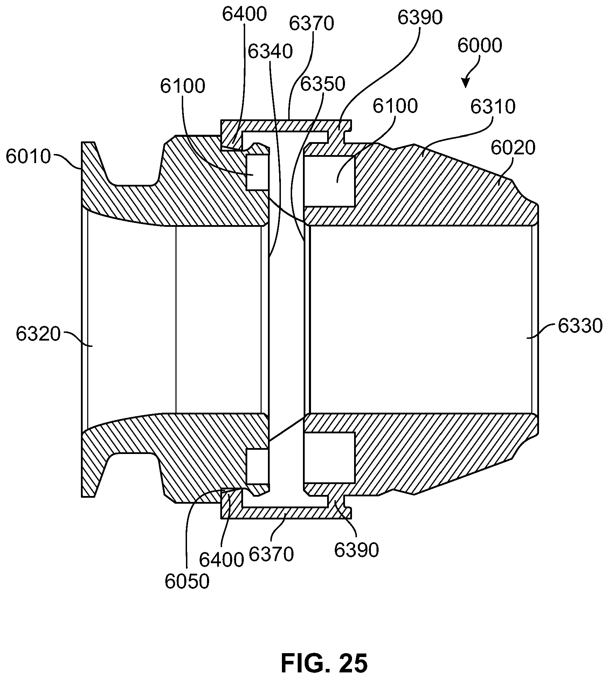

Each arm 6370 is provided with a radially inwardly extending projecting portion 6400, e.g. at a distal end to the mounting portion 6390. The arms 6370 are configured such that when the first and second connector parts 6010, 6020 are brought together the radially inwardly extending projecting portions 6400 can slide over the cylindrical outer wall 6360 of the first connector part 6010, until the radially inwardly extending projecting portions 6400 snap into place in the groove 6050 (see Fig. 25).

In the examples shown in Figs. 14 - 25, two arms 6370 are provided. However, in other examples more than two arms 6370 may be provided. The groove 6050 requires twice as many contiguous substantially helical portions as there are arms provided, e.g. a connector 6000 provided with three arms requires a groove 6050 with six contiguous substantially helical portions having alternating orientations.

In examples, each mounting portion 6390 functions as a resilient living hinge to allow the required radial movement of the radially inwardly extending projecting portions 6400 for engagement of the connector parts 6010, 6020. Alternatively, or additionally, the arms 6370 may flex outwardly when the radially inwardly extending projecting portions 6400 are sliding over the cylindrical outer wall 6360, and may resiliently flex back into position when the inwardly extending projecting portions 6400 engage the groove 6050.

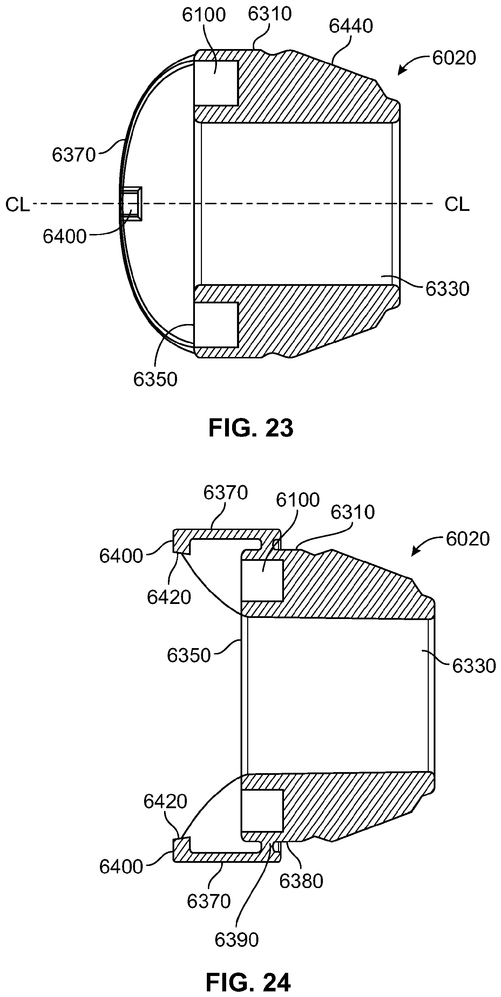

As best seen in Figs. 18 and 19, in examples, a front edge 6410 of the first connector part 6010 may be bevelled to urge the arms 6370 radially outward as the first and second connector parts 6010, 6020 are brought into contact. Additionally, or alternatively, the radially projecting parts 6400 may be provided with a bevelled or ramped inner face 6420 (best seen in Fig. 24) to assist in urging the arms 6370 outward.

In examples of the disclosure, the magnetic attractive force between the magnetic portion and the magnetic and/or ferromagnetic portion is sufficient that when the second connector part 6020 is positioned relative to the first connector part 6010 such that the radially projecting portions 6400 engage any part of the groove 6050 (e.g. as seen in Fig. 25), the magnetic attractive force can move the first and second connector parts to the connected configuration (e.g. as shown in Figs. 14 - 16) in the absence of any external force on the connector parts. However, in examples, the attractive force between the magnetic portions is no more than 10N when the first and second connector parts 6010, 6020 are in the fully engaged position. By providing a relatively weak magnetic attraction between the parts 6100 it is possible to avoid the connector parts 6010, 6020 violently snapping together during assembly.

When in the fully connected configuration, the first and second connector parts 6010, 6020 are held together by both the magnetic attraction between the magnetic portions 6100, and by the engagement of the radially projecting parts 6400 with the groove 6050. In examples, a tension force of at least 20N is required to separate the first and second connector parts 6010, 6020 from the fully engaged position.

In order to separate the connector parts 6010, 6020, one connector part is rotated relative to another. This rotation causes the radially projecting portions 6400 to follow the groove 6050 and to thereby separate the two parts (thereby increasing the separation between the magnetically attracting parts), thus decreasing the magnetic attractive force between the two parts.

With the connectors rotated such that the radially projecting portions 6400 are at the frontmost portion of the groove 6050 (e.g. as shown in Fig. 25) the two connector parts 6010, 6020 can be pulled apart. In the example shown in Figures 14 - 25, the frontmost portion 6430 of the groove 6050 may be provided with a ramped or bevelled portion (see Fig. 18) configured to assist in urging the arms 6370 outward when the connector parts 6010, 6020 are being separated (similar to bevel 6410). However, in other examples (not shown) the frontmost portion of the groove 6050 may be open to the engaging face 6340 the connector part 6010, such that the radially projecting portions 6400 can be removed from the groove 6050 without the need to deflect the arms 6370 outward.

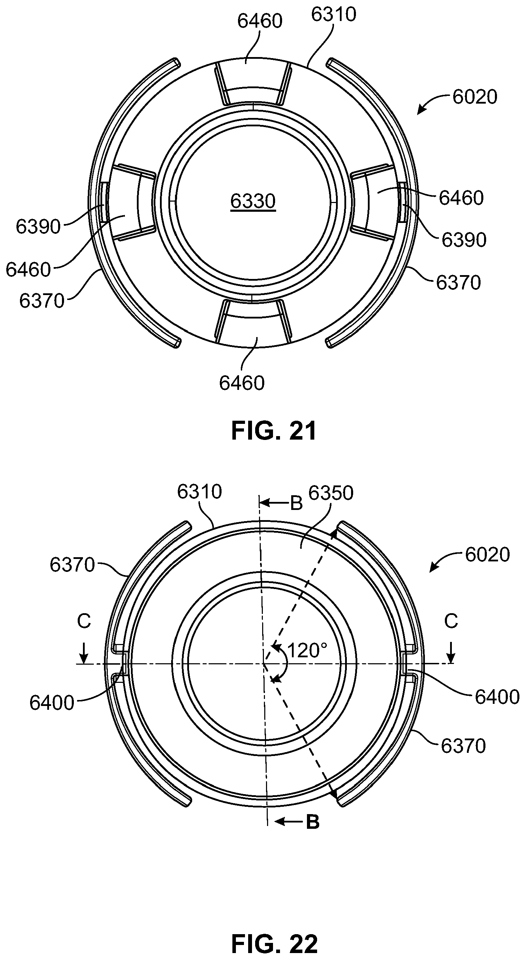

In some forms of the technology, the arms 6370 may be curved about the central axis CL of the passage. In the example shown in Figs 14 - 25, each arm 6370, when viewed from one end, forms a 120 degree constant radius curve, as best seen in Fig. 22. In examples with three arms, each arm, when viewed from one end, may form an 80 degree constant radius curve.

A portion 6440 of the body 6310 of the second connector part 6020 which is distal the first end may taper inwardly. The outer surface 6450 of the inwardly tapered portion 6440 may be provided with a plurality of broad ribs 6460 spaced around the circumference of the body 6310. The ribs 6460 may assist the patient to grip the body and to rotate the body 6310 when disengaging the first connector part 6010 from the second connector part 6020.

In examples (not shown), the second connector part may comprise a first body part rotatably connected to a second body part, similar to the example shown in Figs. 7 to 13.

The first connector part 6010 may be permanently engaged with, or formed integrally with, a patient interface 3000, air circuit 4170, RPT 4000 or humidifier 5000. In examples, the body 6300 of the first connector part 6010 is provided with a circumferential groove for engaging an anterior face of a patient interface. The second connector part 6020 may be engaged with, or formed integrally with an air circuit 4170.

### 4.4 RPT DEVICE

An RPT device 4000 in accordance with one aspect of the present technology comprises mechanical, pneumatic, and/or electrical components and is configured to execute one or more algorithms 4300, such as any of the methods, in whole or in part, described herein. The RPT device 4000 may be configured to generate a flow of air for delivery to a patient's airways, such as to treat one or more of the respiratory conditions described elsewhere in the present document.

In one form, the RPT device 4000 is constructed and arranged to be capable of delivering a flow of air in a range of -20 L/min to +150 L/min while maintaining a positive pressure of at least 6 cmH2O, or at least 10cmH2O, or at least 20 cmH2O.

The RPT device may have an external housing 4010, formed in two parts, an upper portion 4012 and a lower portion 4014. Furthermore, the external housing 4010 may include one or more panel(s) 4015. The RPT device 4000 comprises a chassis 4016 that supports one or more internal components of the RPT device 4000. The RPT device 4000 may include a handle 4018.

The pneumatic path of the RPT device 4000 may comprise one or more air path items, e.g., filters 4110 such as an inlet air filter 4112 and outlet air filter 4114, an inlet muffler 4122, a pressure generator 4140 capable of supplying air at positive pressure (e.g., a blower 4142 comprising a motor 4144), a muffler 4120 such as an outlet muffler 4124 and one or more transducers 4270, such as pressure sensors and flow rate sensors.

One or more of the air path items may be located within a removable unitary structure which will be referred to as a pneumatic block 4020. The pneumatic block 4020 may be located within the external housing 4010. In one form a pneumatic block 4020 is supported by, or formed as part of the chassis 4016.

The RPT device 4000 may have an electrical power supply 4210, one or more input devices, a central controller, a therapy device controller, a pressure generator 4140, one or more protection circuits, memory, transducers 4270, data communication interface and one or more output devices. Electrical components 4200 may be mounted on a single Printed Circuit Board Assembly (PCBA) 4202. In an alternative form, the RPT device 4000 may include more than one PCBA 4202. 4.5 AIR CIRCUIT

An air circuit 4170 in accordance with an aspect of the present technology is a conduit or a tube constructed and arranged to allow, in use, a flow of air to travel between two components such as RPT device 4000 and the patient interface 3000.

In particular, the air circuit 4170 may be in fluid connection with the outlet of the pneumatic block 4020 and the patient interface. The air circuit may be referred to as an air delivery tube. In some cases there may be separate limbs of the circuit for inhalation and exhalation. In other cases a single limb is used.

In some forms, the air circuit 4170 may comprise one or more heating elements configured to heat air in the air circuit, for example to maintain or raise the temperature of the air. The heating element may be in a form of a heated wire circuit, and may comprise one or more transducers, such as temperature sensors. In one form, the heated wire circuit may be helically wound around the axis of the air circuit 4170. The heating element may be in communication with a controller such as a central controller 4230. One example of an air circuit 4170 comprising a heated wire circuit is described in United States Patent 8,733,349.

### 4.5.1 Supplementary gas delivery

In one form of the present technology, supplementary gas, e.g. oxygen, 4180 is delivered to one or more points in the pneumatic path, such as upstream of the pneumatic block 4020, to the air circuit 4170, and/or to the patient interface 3000.

### 4.6 HUMIDIFIER

### 4.6.1 Humidifier overview

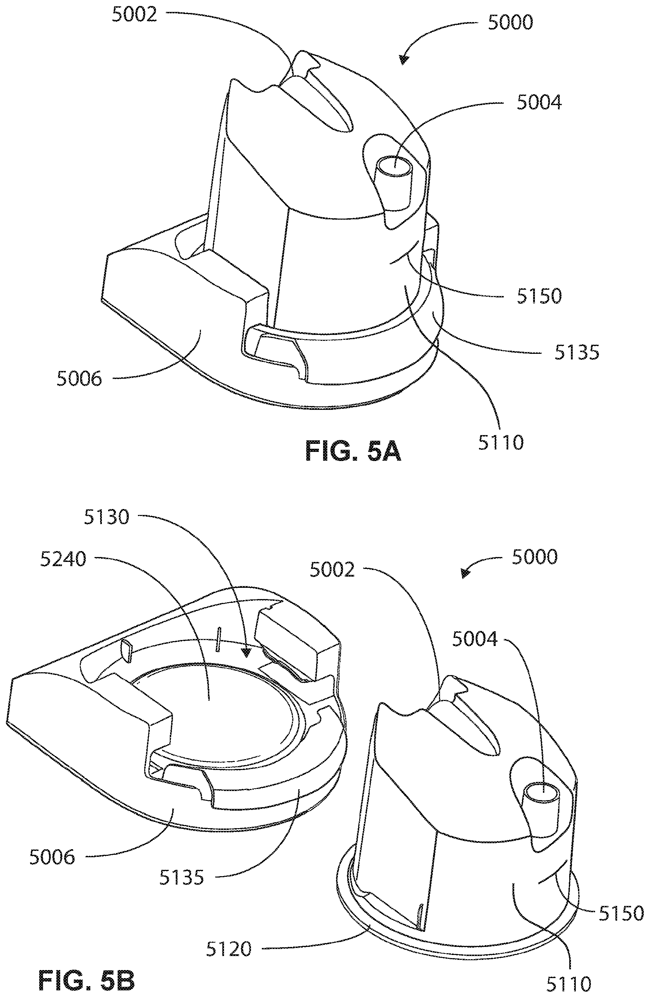

In one form of the present technology there is provided a humidifier 5000 (e.g. as shown in Fig. 5A) to change the absolute humidity of air or gas for delivery to a patient relative to ambient air. Typically, the humidifier 5000 is used to increase the absolute humidity and increase the temperature of the flow of air (relative to ambient air) before delivery to the patient's airways.

The humidifier 5000 may comprise a humidifier reservoir 5110, a humidifier inlet 5002 to receive a flow of air, and a humidifier outlet 5004 to deliver a humidified flow of *air.* In some forms, as shown in Fig. 5A and Fig. 5B, an inlet and an outlet of the humidifier reservoir 5110 may be the humidifier inlet 5002 and the humidifier outlet 5004 respectively. The humidifier 5000 may further comprise a humidifier base 5006, which may be adapted to receive the humidifier reservoir 5110 and comprise a heating element 5240.

In examples the humidifier reservoir further comprises a conductive portion 5120, a locking lever 5135 and a water level indicator 5150.

In one form of the present technology, an anti-spill back valve 4160 is located between the humidifier 5000 and the pneumatic block 4020.

### 4.7 BREATHING WAVEFORMS

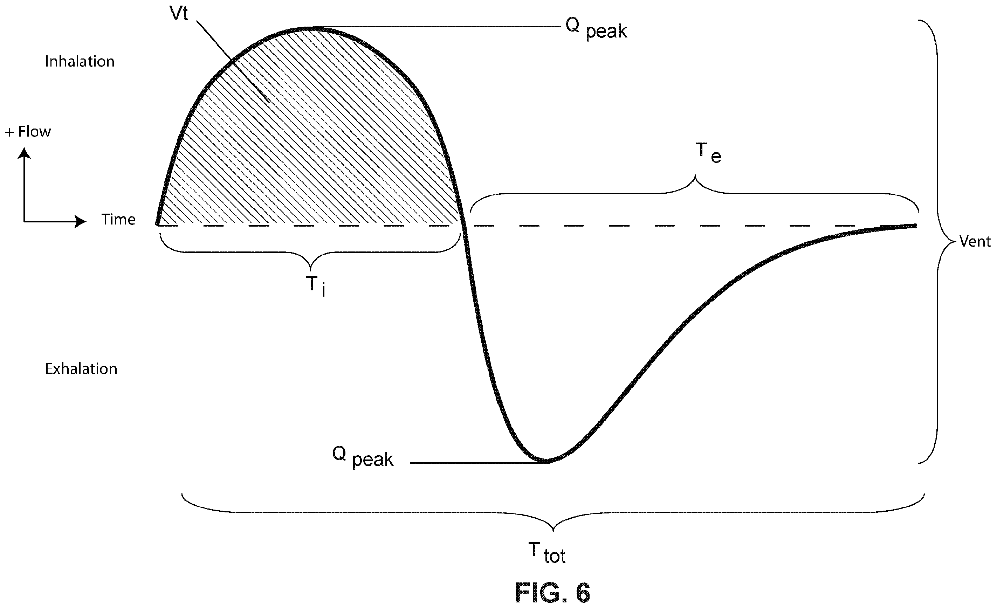

Fig. 6 shows a model typical breath waveform of a person while sleeping. The horizontal axis is time, and the vertical axis is respiratory flow rate. While the parameter values may vary, a typical breath may have the following approximate values: tidal volume *Vt* 0.5L, inhalation time *Ti* 1.6s, peak inspiratory flow rate *Qpeak* 0.4 L/s, exhalation time *Te* 2.4s, peak expiratory flow rate *Qpeak* -0.5 L/s. The total duration of the breath, *Ttot,* is about 4s. The person typically breathes at a rate of about 15 breaths per minute (BPM), with Ventilation *Vent* about 7.5 L/min. A typical duty cycle, the ratio of *Ti* to *Ttot,* is about 40%.

### 4.8 RESPIRATORY THERAPY MODES

Various respiratory therapy modes may be implemented by the disclosed respiratory therapy system.

### 4.9 GLOSSARY

For the purposes of the present technology disclosure, in certain forms of the present technology, one or more of the following definitions may apply. In other forms of the present technology, alternative definitions may apply.

### 4.9.1 General

*Air*: In certain forms of the present technology, air may be taken to mean atmospheric air, and in other forms of the present technology air may be taken to mean some other combination of breathable gases, e.g. oxygen enriched air.

*Ambient*: In certain forms of the present technology, the term ambient will be taken to mean (i) external of the treatment system or patient, and (ii) immediately surrounding the treatment system or patient.

For example, ambient humidity with respect to a humidifier may be the humidity of air immediately surrounding the humidifier, e.g. the humidity in the room where a patient is sleeping. Such ambient humidity may be different to the humidity outside the room where a patient is sleeping.

In another example, ambient pressure may be the pressure immediately surrounding or external to the body.

In certain forms, ambient (e.g., acoustic) noise may be considered to be the background noise level in the room where a patient is located, other than for example, noise generated by an RPT device or emanating from a mask or patient interface. Ambient noise may be generated by sources outside the room.

*Automatic Positive Airway Pressure (APAP) therapy:* CPAP therapy in which the treatment pressure is automatically adjustable, e.g. from breath to breath, between minimum and maximum limits, depending on the presence or absence of indications of SDB events.

*Continuous Positive Airway Pressure (CPAP) therapy:* Respiratory pressure therapy in which the treatment pressure is approximately constant through a respiratory cycle of a patient. In some forms, the pressure at the entrance to the airways will be slightly higher during exhalation, and slightly lower during inhalation. In some forms, the pressure will vary between different respiratory cycles of the patient, for example, being increased in response to detection of indications of partial upper airway obstruction, and decreased in the absence of indications of partial upper airway obstruction.

*Flow rate:* The volume (or mass) of air delivered per unit time. Flow rate may refer to an instantaneous quantity. In some cases, a reference to flow rate will be a reference to a scalar quantity, namely a quantity having magnitude only. In other cases, a reference to flow rate will be a reference to a vector quantity, namely a quantity having both magnitude and direction. Flow rate may be given the symbol Q. 'Flow rate' is sometimes shortened to simply 'flow' or 'airflow'.

In the example of patient respiration, a flow rate may be nominally positive for the inspiratory portion of a breathing cycle of a patient, and hence negative for the expiratory portion of the breathing cycle of a patient. Device flow rate, *Qd,* is the flow rate of air leaving the RPT device. Total flow rate, *Qt,* is the flow rate of air and any supplementary gas reaching the patient interface via the air circuit. Vent flow rate, *Qv,* is the flow rate of air leaving a vent to allow washout of exhaled gases. Leak flow rate, *Ql,* is the flow rate of leak from a patient interface system or elsewhere. Respiratory flow rate, *Qr,* is the flow rate of air that is received into the patient's respiratory system.

*Flow therapy:* Respiratory therapy comprising the delivery of a flow of air to an entrance to the airways at a controlled flow rate referred to as the treatment flow rate that is typically positive throughout the patient's breathing cycle.

*Humidifier:* The word humidifier will be taken to mean a humidifying apparatus constructed and arranged, or configured with a physical structure to be capable of providing a therapeutically beneficial amount of water (H₂O) vapour to a flow of air to ameliorate a medical respiratory condition of a patient.

*Leak:* The word leak will be taken to be an unintended flow of air. In one example, leak may occur as the result of an incomplete seal between a mask and a patient's face. In another example leak may occur in a swivel elbow to the ambient.

*Noise, conducted (acoustic):* Conducted noise in the present document refers to noise which is carried to the patient by the pneumatic path, such as the air circuit and the patient interface as well as the air therein. In one form, conducted noise may be quantified by measuring sound pressure levels at the end of an air circuit.

*Noise, radiated (acoustic):* Radiated noise in the present document refers to noise which is carried to the patient by the ambient air. In one form, radiated noise may be quantified by measuring sound power/pressure levels of the object in question according to ISO 3744.

*Noise, vent (acoustic):* Vent noise in the present document refers to noise which is generated by the flow of air through any vents such as vent holes of the patient interface.

*Oxygen enriched air*: Air with a concentration of oxygen greater than that of atmospheric air (21%), for example at least about 50% oxygen, at least about 60% oxygen, at least about 70% oxygen, at least about 80% oxygen, at least about 90% oxygen, at least about 95% oxygen, at least about 98% oxygen, or at least about 99% oxygen. "Oxygen enriched air" is sometimes shortened to "oxygen".

*Medical Oxygen:* Medical oxygen is defined as oxygen enriched air with an oxygen concentration of 80% or *greater. Patient:* A person, whether or not they are suffering from a respiratory condition.

*Pressure:* Force per unit area. Pressure may be expressed in a range of units, including cmH₂O, g-f/cm² and hectopascal. 1 cmH₂O is equal to 1 g-f/cm² and is approximately 0.98 hectopascal (1 hectopascal = 100 Pa = 100 N/m² = 1 millibar ~ 0.001 atm). In this specification, unless otherwise stated, pressure is given in units of cmH₂O.

The pressure in the patient interface is given the symbol *Pm,* while the treatment pressure, which represents a target value to be achieved by the interface pressure *Pm* at the current instant of time, is given the symbol *Pt.*

*Respiratory Pressure Therapy:* The application of a supply of air to an entrance to the airways at a treatment pressure that is typically positive with respect to atmosphere.

*Ventilator:* A mechanical device that provides pressure support to a patient to perform some or all of the work of breathing.

### 4.9.1.1 Materials

*Silicone or Silicone Elastomer:* A synthetic rubber. In this specification, a reference to silicone is a reference to liquid silicone rubber (LSR) or a compression moulded silicone rubber (CMSR). One form of commercially available LSR is SILASTIC (included in the range of products sold under this trademark), manufactured by Dow Corning. Another manufacturer of LSR is Wacker. Unless otherwise specified to the contrary, an exemplary form of LSR has a Shore A (or Type A) indentation hardness in the range of about 35 to about 45 as measured using ASTM D2240.

*Polycarbonate:* a thermoplastic polymer of Bisphenol-A Carbonate.

### 4.9.1.2 Mechanical properties

Resilience: Ability of a material to absorb energy when deformed elastically and to release the energy upon unloading.

Resilient: Will release substantially all of the energy when unloaded. Includes e.g. certain silicones, and thermoplastic elastomers.

Hardness: The ability of a material per se to resist deformation (e.g. described by a Young's Modulus, or an indentation hardness scale measured on a standardised sample size).
- 'Soft' materials may include silicone or thermo-plastic elastomer (TPE), and may, e.g. readily deform under finger pressure.
- 'Hard' materials may include polycarbonate, polypropylene, steel or aluminium, and may not e.g. readily deform under finger pressure.

Stiffness (or rigidity) of a structure or component: The ability of the structure or component to resist deformation in response to an applied load. The load may be a force or a moment, e.g. compression, tension, bending or torsion. The structure or component may offer different resistances in different directions. The inverse of stiffness is flexibility.

Floppy structure or component: A structure or component that will change shape, e.g. bend, when caused to support its own weight, within a relatively short period of time such as 1 second.

Rigid structure or component: A structure or component that will not substantially change shape when subject to the loads typically encountered in use. An example of such a use may be setting up and maintaining a patient interface in sealing relationship with an entrance to a patient's airways, e.g. at a load of approximately 20 to 30 cmH2O pressure.

As an example, an I-beam may comprise a different bending stiffness (resistance to a bending load) in a first direction in comparison to a second, orthogonal direction. In another example, a structure or component may be floppy in a first direction and rigid in a second direction.

### 4.9.2 Respiratory cycle

Apnea: According to some definitions, an apnea is said to have occurred when flow falls below a predetermined threshold for a duration, e.g. 10 seconds. An obstructive apnea will be said to have occurred when, despite patient effort, some obstruction of the airway does not allow air to flow. A central apnea will be said to have occurred when an apnea is detected that is due to a reduction in breathing effort, or the absence of breathing effort, despite the airway being patent. A mixed apnea occurs when a reduction or absence of breathing effort coincides with an obstructed airway.

Breathing rate: The rate of spontaneous respiration of a patient, usually measured in breaths per minute.

Duty cycle: The ratio of inhalation time, Ti to total breath time, Ttot.

Effort (breathing): The work done by a spontaneously breathing person attempting to breathe.

Expiratory portion of a breathing cycle: The period from the start of expiratory flow to the start of inspiratory flow.

Flow limitation: Flow limitation will be taken to be the state of affairs in a patient's respiration where an increase in effort by the patient does not give rise to a corresponding increase in flow. Where flow limitation occurs during an inspiratory portion of the breathing cycle it may be described as inspiratory flow limitation. Where flow limitation occurs during an expiratory portion of the breathing cycle it may be described as expiratory flow limitation.

Types of flow limited inspiratory waveforms:
(i) Flattened: Having a rise followed by a relatively flat portion, followed by a fall.
(ii) M-shaped: Having two local peaks, one at the leading edge, and one at the trailing edge, and a relatively flat portion between the two peaks.
(iii) Chair-shaped: Having a single local peak, the peak being at the leading edge, followed by a relatively flat portion.
(iv) Reverse-chair shaped: Having a relatively flat portion followed by single local peak, the peak being at the trailing edge.

Hypopnea: According to some definitions, a hypopnea is taken to be a reduction in flow, but not a cessation of flow. In one form, a hypopnea may be said to have occurred when there is a reduction in flow below a threshold rate for a duration. A central hypopnea will be said to have occurred when a hypopnea is detected that is due to a reduction in breathing effort. In one form in adults, either of the following may be regarded as being hypopneas:
(i) a 30% reduction in patient breathing for at least 10 seconds plus an associated 4% desaturation; or
(ii) a reduction in patient breathing (but less than 50%) for at least 10 seconds, with an associated desaturation of at least 3% or an arousal.

Hyperpnea: An increase in flow to a level higher than normal.

Inspiratory portion of a breathing cycle: The period from the start of inspiratory flow to the start of expiratory flow will be taken to be the inspiratory portion of a breathing cycle.

Patency (airway): The degree of the airway being open, or the extent to which the airway is open. A patent airway is open. Airway patency may be quantified, for example with a value of one (1) being patent, and a value of zero (0), being closed (obstructed).

Positive End-Expiratory Pressure (PEEP): The pressure above atmosphere in the lungs that exists at the end of expiration.

Peak flow rate (Qpeak): The maximum value of flow rate during the inspiratory portion of the respiratory flow waveform.

Respiratory flow rate, patient airflow rate, respiratory airflow rate (Qr): These terms may be understood to refer to the RPT device's estimate of respiratory flow rate, as opposed to "true respiratory flow rate" or "true respiratory flow rate", which is the actual respiratory flow rate experienced by the patient, usually expressed in litres per minute.

Tidal volume (Vt): The volume of air inhaled or exhaled during normal breathing, when extra effort is not applied. In principle the inspiratory volume Vi (the volume of air inhaled) is equal to the expiratory volume Ve (the volume of air exhaled), and therefore a single tidal volume Vt may be defined as equal to either quantity. In practice the tidal volume Vt is estimated as some combination, e.g. the mean, of the inspiratory volume Vi and the expiratory volume Ve.

Inhalation Time (Ti): The duration of the inspiratory portion of the respiratory flow rate waveform.

Exhalation Time (Te): The duration of the expiratory portion of the respiratory flow rate waveform.

Total Time (Ttot): The total duration between the start of one inspiratory portion of a respiratory flow rate waveform and the start of the following inspiratory portion of the respiratory flow rate waveform.

Typical recent ventilation: The value of ventilation around which recent values of ventilation Vent over some predetermined timescale tend to cluster, that is, a measure of the central tendency of the recent values of ventilation.

Upper airway obstruction (UAO): includes both partial and total upper airway obstruction. This may be associated with a state of flow limitation, in which the flow rate increases only slightly or may even decrease as the pressure difference across the upper airway increases (Starling resistor behaviour).

Ventilation (Vent): A measure of a rate of gas being exchanged by the patient's respiratory system. Measures of ventilation may include one or both of inspiratory and expiratory flow, per unit time. When expressed as a volume per minute, this quantity is often referred to as "minute ventilation". Minute ventilation is sometimes given simply as a volume, understood to be the volume per minute.

### 4.9.3 Ventilation

Adaptive Servo-Ventilator (ASV): A servo-ventilator that has a changeable, rather than fixed target ventilation. The changeable target ventilation may be learned from some characteristic of the patient, for example, a respiratory characteristic of the patient.

Backup rate: A parameter of a ventilator that establishes the minimum breathing rate (typically in number of breaths per minute) that the ventilator will deliver to the patient, if not triggered by spontaneous respiratory effort.

Cycled: The termination of a ventilator's inspiratory phase. When a ventilator delivers a breath to a spontaneously breathing patient, at the end of the inspiratory portion of the breathing cycle, the ventilator is said to be cycled to stop delivering the breath.

Expiratory positive airway pressure (EPAP): a base pressure, to which a pressure varying within the breath is added to produce the desired interface pressure which the ventilator will attempt to achieve at a given time.

End expiratory pressure (EEP): Desired interface pressure which the ventilator will attempt to achieve at the end of the expiratory portion of the breath. If the pressure waveform template Π(Φ) is zero-valued at the end of expiration, i.e. Π(Φ) = 0 when Φ = 1, the EEP is equal to the EPAP.

Inspiratory positive airway pressure (IPAP): Maximum desired interface pressure which the ventilator will attempt to achieve during the inspiratory portion of the breath.

Pressure support: A number that is indicative of the increase in pressure during ventilator inspiration over that during ventilator expiration, and generally means the difference in pressure between the maximum value during inspiration and the base pressure (e.g., PS = IPAP - EPAP). In some contexts, pressure support means the difference which the ventilator aims to achieve, rather than what it actually achieves.

Servo-ventilator: A ventilator that measures patient ventilation, has a target ventilation, and which adjusts the level of pressure support to bring the patient ventilation towards the target ventilation.

Spontaneous/Timed (S/T): A mode of a ventilator or other device that attempts to detect the initiation of a breath of a spontaneously breathing patient. If however, the device is unable to detect a breath within a predetermined period of time, the device will automatically initiate delivery of the breath.

Swing: Equivalent term to pressure support.

Triggered: When a ventilator, or other respiratory therapy device such as an RPT device or portable oxygen concentrator, delivers a volume of breathable gas to a spontaneously breathing patient, it is said to be triggered to do so. Triggering usually takes place at or near the initiation of the respiratory portion of the breathing cycle by the patient's efforts.

### 4.9.4 Anatomy

### 4.9.4.1 Anatomy of the face

Ala: the external outer wall or "wing" of each nostril (plural: alar)

Alare: The most lateral point on the nasal ala.

Alar curvature (or alar crest) point: The most posterior point in the curved base line of each ala, found in the crease formed by the union of the ala with the cheek.

Auricle: The whole external visible part of the ear.

(nose) Bony framework: The bony framework of the nose comprises the nasal bones, the frontal process of the maxillae and the nasal part of the frontal bone.

(nose) Cartilaginous framework: The cartilaginous framework of the nose comprises the septal, lateral, major and minor cartilages.

Columella: the strip of skin that separates the nares and which runs from the pronasale to the upper lip.

Columella angle: The angle between the line drawn through the midpoint of the nostril aperture and a line drawn perpendicular to the Frankfort horizontal while intersecting subnasale.

Frankfort horizontal plane: A line extending from the most inferior point of the orbital margin to the left tragion. The tragion is the deepest point in the notch superior to the tragus of the auricle.

Glabella: Located on the soft tissue, the most prominent point in the midsagittal plane of the forehead.

Lateral nasal cartilage: A generally triangular plate of cartilage. Its superior margin is attached to the nasal bone and frontal process of the maxilla, and its inferior margin is connected to the greater alar cartilage.

Lip, lower (labrale inferius):
Lip, upper (labrale superius):
Greater alar cartilage: A plate of cartilage lying below the lateral nasal cartilage. It is curved around the anterior part of the naris. Its posterior end is connected to the frontal process of the maxilla by a tough fibrous membrane containing three or four minor cartilages of the ala.

Nares (Nostrils): Approximately ellipsoidal apertures forming the entrance to the nasal cavity. The singular form of nares is naris (nostril). The nares are separated by the nasal septum.

Naso-labial sulcus or Naso-labial fold: The skin fold or groove that runs from each side of the nose to the corners of the mouth, separating the cheeks from the upper lip.

Naso-labial angle: The angle between the columella and the upper lip, while intersecting subnasale.

Otobasion inferior: The lowest point of attachment of the auricle to the skin of the face.

Otobasion superior: The highest point of attachment of the auricle to the skin of the face.

Pronasale: the most protruded point or tip of the nose, which can be identified in lateral view of the rest of the portion of the head.

Philtrum: the midline groove that runs from lower border of the nasal septum to the top of the lip in the upper lip region.

Pogonion: Located on the soft tissue, the most anterior midpoint of the chin.

Ridge (nasal): The nasal ridge is the midline prominence of the nose, extending from the Sellion to the Pronasale.

Sagittal plane: A vertical plane that passes from anterior (front) to posterior (rear). The midsagittal plane is a sagittal plane that divides the body into right and left halves.

Sellion: Located on the soft tissue, the most concave point overlying the area of the frontonasal suture.

Septal cartilage (nasal): The nasal septal cartilage forms part of the septum and divides the front part of the nasal cavity.

Subalare: The point at the lower margin of the alar base, where the alar base joins with the skin of the superior (upper) lip.

Subnasal point: Located on the soft tissue, the point at which the columella merges with the upper lip in the midsagittal plane.

Supramenton: The point of greatest concavity in the midline of the lower lip between labrale inferius and soft tissue pogonion

### 4.9.4.2 Anatomy of the skull

Frontal bone: The frontal bone includes a large vertical portion, the squama frontalis, corresponding to the region known as the forehead.

Mandible: The mandible forms the lower jaw. The mental protuberance is the bony protuberance of the jaw that forms the chin.

Maxilla: The maxilla forms the upper jaw and is located above the mandible and below the orbits. The frontal process of the maxilla projects upwards by the side of the nose, and forms part of its lateral boundary.

Nasal bones: The nasal bones are two small oblong bones, varying in size and form in different individuals; they are placed side by side at the middle and upper part of the face, and form, by their junction, the "bridge" of the nose.

Nasion: The intersection of the frontal bone and the two nasal bones, a depressed area directly between the eyes and superior to the bridge of the nose.

Occipital bone: The occipital bone is situated at the back and lower part of the cranium. It includes an oval aperture, the foramen magnum, through which the cranial cavity communicates with the vertebral canal. The curved plate behind the foramen magnum is the squama occipitalis.

Orbit: The bony cavity in the skull to contain the eyeball.

Parietal bones: The parietal bones are the bones that, when joined together, form the roof and sides of the cranium.

Temporal bones: The temporal bones are situated on the bases and sides of the skull, and support that part of the face known as the temple.

Zygomatic bones: The face includes two zygomatic bones, located in the upper and lateral parts of the face and forming the prominence of the cheek.

### 4.9.4.3 Anatomy of the respiratory system

Diaphragm: A sheet of muscle that extends across the bottom of the rib cage. The diaphragm separates the thoracic cavity, containing the heart, lungs and ribs, from the abdominal cavity. As the diaphragm contracts the volume of the thoracic cavity increases and air is drawn into the lungs.

Larynx: The larynx, or voice box houses the vocal folds and connects the inferior part of the pharynx (hypopharynx) with the trachea.

Lungs: The organs of respiration in humans. The conducting zone of the lungs contains the trachea, the bronchi, the bronchioles, and the terminal bronchioles. The respiratory zone contains the respiratory bronchioles, the alveolar ducts, and the alveoli.

Nasal cavity: The nasal cavity (or nasal fossa) is a large air filled space above and behind the nose in the middle of the face. The nasal cavity is divided in two by a vertical fin called the nasal septum. On the sides of the nasal cavity are three horizontal outgrowths called nasal conchae (singular "concha") or turbinates. To the front of the nasal cavity is the nose, while the back blends, via the choanae, into the nasopharynx.

Pharynx: The part of the throat situated immediately inferior to (below) the nasal cavity, and superior to the oesophagus and larynx. The pharynx is conventionally divided into three sections: the nasopharynx (epipharynx) (the nasal part of the pharynx), the oropharynx (mesopharynx) (the oral part of the pharynx), and the laryngopharynx (hypopharynx).

### 4.9.5 Patient interface

Anti-asphyxia valve (AAV): The component or sub-assembly of a mask system that, by opening to atmosphere in a failsafe manner, reduces the risk of excessive CO2 rebreathing by a patient.

Elbow: An elbow is an example of a structure that directs an axis of flow of air travelling therethrough to change direction through an angle. In one form, the angle may be approximately 90 degrees. In another form, the angle may be more, or less than 90 degrees. The elbow may have an approximately circular cross-section. In another form the elbow may have an oval or a rectangular cross-section. In certain forms an elbow may be rotatable with respect to a mating component, e.g. about 360 degrees. In certain forms an elbow may be removable from a mating component, e.g. via a snap connection. In certain forms, an elbow may be assembled to a mating component via a one-time snap during manufacture, but not removable by a patient.

Frame: Frame will be taken to mean a mask structure that bears the load of tension between two or more points of connection with a headgear. A mask frame may be a non-airtight load bearing structure in the mask. However, some forms of mask frame may also be air-tight.

Headgear: Headgear will be taken to mean a form of positioning and stabilizing structure designed for use on a head. For example the headgear may comprise a collection of one or more struts, ties and stiffeners configured to locate and retain a patient interface in position on a patient's face for delivery of respiratory therapy. Some ties are formed of a soft, flexible, elastic material such as a laminated composite of foam and fabric.

Membrane: Membrane will be taken to mean a typically thin element that has, preferably, substantially no resistance to bending, but has resistance to being stretched.

Plenum chamber: a mask plenum chamber will be taken to mean a portion of a patient interface having walls at least partially enclosing a volume of space, the volume having air therein pressurised above atmospheric pressure in use. A shell may form part of the walls of a mask plenum chamber.

Seal: May be a noun form ("a seal") which refers to a structure, or a verb form ("to seal") which refers to the effect. Two elements may be constructed and/or arranged to 'seal' or to effect 'sealing' therebetween without requiring a separate 'seal' element per se.

Shell: A shell will be taken to mean a curved, relatively thin structure having bending, tensile and compressive stiffness. For example, a curved structural wall of a mask may be a shell. In some forms, a shell may be faceted. In some forms a shell may be airtight. In some forms a shell may not be airtight.

Stiffener: A stiffener will be taken to mean a structural component designed to increase the bending resistance of another component in at least one direction.

Strut: A strut will be taken to be a structural component designed to increase the compression resistance of another component in at least one direction.

Swivel (noun): A subassembly of components configured to rotate about a common axis, preferably independently, preferably under low torque. In one form, the swivel may be constructed to rotate through an angle of at least 360 degrees. In another form, the swivel may be constructed to rotate through an angle less than 360 degrees. When used in the context of an air delivery conduit, the sub-assembly of components preferably comprises a matched pair of cylindrical conduits. There may be little or no leak flow of air from the swivel in use.

Tie (noun): A structure designed to resist tension.

Vent: (noun): A structure that allows a flow of air from an interior of the mask, or conduit, to ambient air for clinically effective washout of exhaled gases. For example, a clinically effective washout may involve a flow rate of about 10 litres per minute to about 100 litres per minute, depending on the mask design and treatment pressure.

### 4.9.6 Shape of structures

Products in accordance with the present technology may comprise one or more three-dimensional mechanical structures, for example a mask cushion or an impeller. The three-dimensional structures may be bounded by two-dimensional surfaces. These surfaces may be distinguished using a label to describe an associated surface orientation, location, function, or some other characteristic. For example a structure may comprise one or more of an anterior surface, a posterior surface, an interior surface and an exterior surface. In another example, a seal-forming structure may comprise a face-contacting (e.g. outer) surface, and a separate non-face-contacting (e.g. underside or inner) surface. In another example, a structure may comprise a first surface and a second surface.

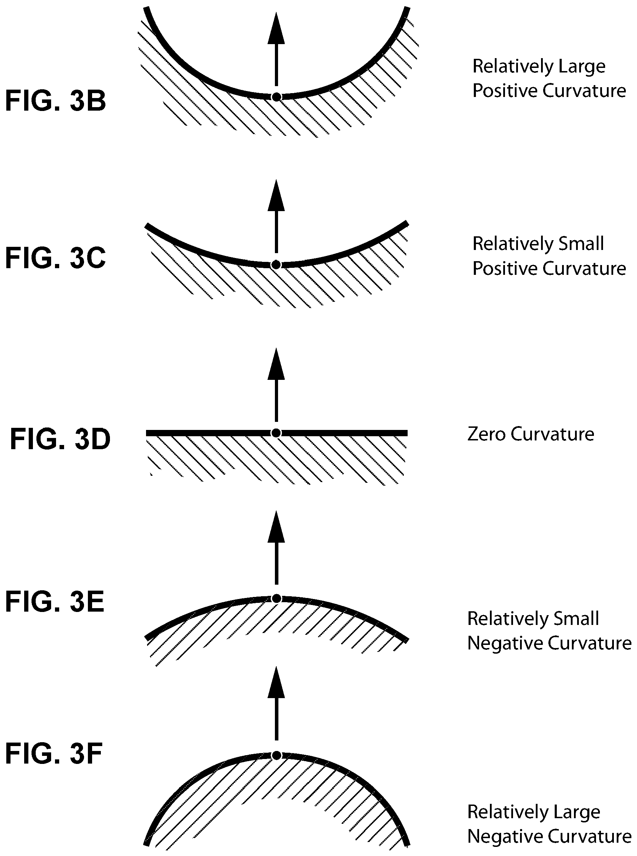

To facilitate describing the shape of the three-dimensional structures and the surfaces, we first consider a cross-section through a surface of the structure at a point, p. See Fig. 3B to Fig. 3F, which illustrate examples of cross-sections at point p on a surface, and the resulting plane curves. Figs. 3B to 3F also illustrate an outward normal vector at p. The outward normal vector at p points away from the surface. In some examples we describe the surface from the point of view of an imaginary small person standing upright on the surface.

### 4.9.6.1 Curvature in one dimension

The curvature of a plane curve at p may be described as having a sign (e.g. positive, negative) and a magnitude (e.g. 1/radius of a circle that just touches the curve at p).

Positive curvature: If the curve at p turns towards the outward normal, the curvature at that point will be taken to be positive (if the imaginary small person leaves the point p they must walk uphill). See Fig. 3B (relatively large positive curvature compared to Fig. 3C) and Fig. 3C (relatively small positive curvature compared to Fig. 3B). Such curves are often referred to as concave.

Zero curvature: If the curve at p is a straight line, the curvature will be taken to be zero (if the imaginary small person leaves the point p, they can walk on a level, neither up nor down). See Fig. 3D.

Negative curvature: If the curve at p turns away from the outward normal, the curvature in that direction at that point will be taken to be negative (if the imaginary small person leaves the point p they must walk downhill). See Fig. 3E (relatively small negative curvature compared to Fig. 3F) and Fig. 3F (relatively large negative curvature compared to Fig. 3E). Such curves are often referred to as convex.

### 4.9.6.2 Curvature of two dimensional surfaces

A description of the shape at a given point on a two-dimensional surface in accordance with the present technology may include multiple normal cross-sections. The multiple cross-sections may cut the surface in a plane that includes the outward normal (a "normal plane"), and each cross-section may be taken in a different direction. Each cross-section results in a plane curve with a corresponding curvature. The different curvatures at that point may have the same sign, or a different sign. Each of the curvatures at that point has a magnitude, e.g. relatively small. The plane curves in Figs. 3B to 3F could be examples of such multiple cross-sections at a particular point.

Principal curvatures and directions: The directions of the normal planes where the curvature of the curve takes its maximum and minimum values are called the principal directions. In the examples of Fig. 3B to Fig. 3F, the maximum curvature occurs in Fig. 3B, and the minimum occurs in Fig. 3F, hence Fig. 3B and Fig. 3F are cross sections in the principal directions. The principal curvatures at p are the curvatures in the principal directions.

Region of a surface: A connected set of points on a surface. The set of points in a region may have similar characteristics, e.g. curvatures or signs.

Saddle region: A region where at each point, the principal curvatures have opposite signs, that is, one is positive, and the other is negative (depending on the direction to which the imaginary person turns, they may walk uphill or downhill).

Dome region: A region where at each point the principal curvatures have the same sign, e.g. both positive (a "concave dome") or both negative (a "convex dome").

Cylindrical region: A region where one principal curvature is zero (or, for example, zero within manufacturing tolerances) and the other principal curvature is non-zero.

Planar region: A region of a surface where both of the principal curvatures are zero (or, for example, zero within manufacturing tolerances).

Edge of a surface: A boundary or limit of a surface or region.

Path: In certain forms of the present technology, 'path' will be taken to mean a path in the mathematical - topological sense, e.g. a continuous space curve from f(0) to f(1) on a surface. In certain forms of the present technology, a 'path' may be described as a route or course, including e.g. a set of points on a surface. (The path for the imaginary person is where they walk on the surface, and is analogous to a garden path).

Path length: In certain forms of the present technology, 'path length' will be taken to mean the distance along the surface from f(0) to f(1), that is, the distance along the path on the surface. There may be more than one path between two points on a surface and such paths may have different path lengths. (The path length for the imaginary person would be the distance they have to walk on the surface along the path).

Straight-line distance: The straight-line distance is the distance between two points on a surface, but without regard to the surface. On planar regions, there would be a path on the surface having the same path length as the straight-line distance between two points on the surface. On non-planar surfaces, there may be no paths having the same path length as the straight-line distance between two points. (For the imaginary person, the straight-line distance would correspond to the distance 'as the crow flies'.)

### 4.9.6.3 Space curves

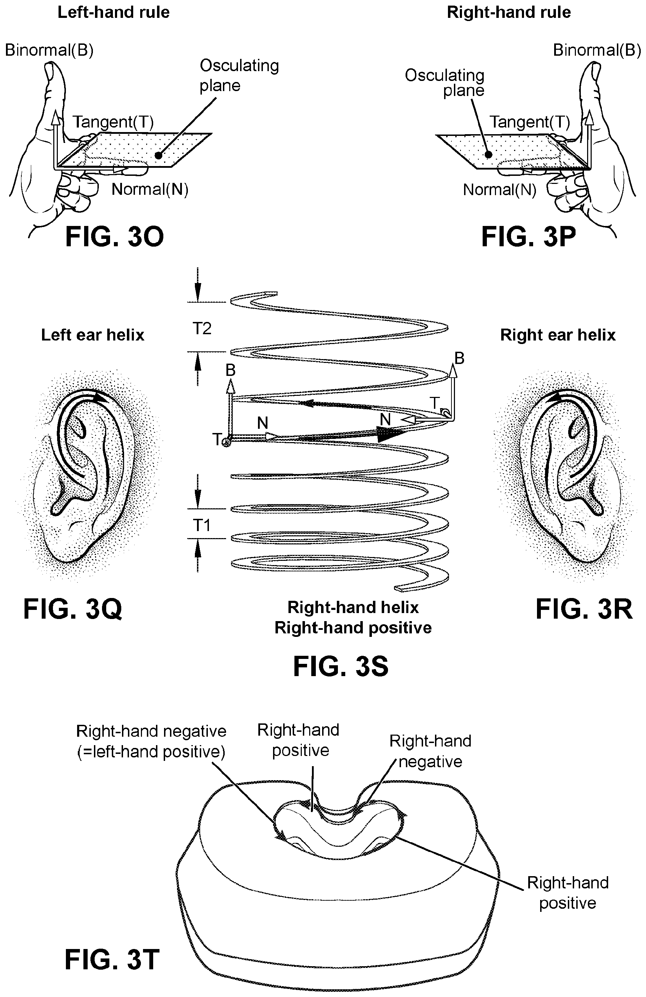

Space curves: Unlike a plane curve, a space curve does not necessarily lie in any particular plane. A space curve may be closed, that is, having no endpoints. A space curve may be considered to be a one-dimensional piece of three-dimensional space. An imaginary person walking on a strand of the DNA helix walks along a space curve. A typical human left ear comprises a helix, which is a left-hand helix, see Fig. 3Q. A typical human right ear comprises a helix, which is a right-hand helix, see Fig. 3R. Fig. 3S shows a right-hand helix. The edge of a structure, e.g. the edge of a membrane or impeller, may follow a space curve. In general, a space curve may be described by a curvature and a torsion at each point on the space curve. Torsion is a measure of how the curve turns out of a plane. Torsion has a sign and a magnitude. The torsion at a point on a space curve may be characterised with reference to the tangent, normal and binormal vectors at that point.

Tangent unit vector (or unit tangent vector): For each point on a curve, a vector at the point specifies a direction from that point, as well as a magnitude. A tangent unit vector is a unit vector pointing in the same direction as the curve at that point. If an imaginary person were flying along the curve and fell off her vehicle at a particular point, the direction of the tangent vector is the direction she would be travelling.

Unit normal vector: As the imaginary person moves along the curve, this tangent vector itself changes. The unit vector pointing in the same direction that the tangent vector is changing is called the unit principal normal vector. It is perpendicular to the tangent vector.

Binormal unit vector: The binormal unit vector is perpendicular to both the tangent vector and the principal normal vector. Its direction may be determined by a right-hand rule (see e.g. Fig. 3P), or alternatively by a left-hand rule (Fig. 3O).

Osculating plane: The plane containing the unit tangent vector and the unit principal normal vector. See Figures 3O and 3P.

Torsion of a space curve: The torsion at a point of a space curve is the magnitude of the rate of change of the binormal unit vector at that point. It measures how much the curve deviates from the osculating plane. A space curve which lies in a plane has zero torsion. A space curve which deviates a relatively small amount from the osculating plane will have a relatively small magnitude of torsion (e.g. a gently sloping helical path). A space curve which deviates a relatively large amount from the osculating plane will have a relatively large magnitude of torsion (e.g. a steeply sloping helical path). With reference to Fig. 3S, since T2>T1, the magnitude of the torsion near the top coils of the helix of Fig. 3S is greater than the magnitude of the torsion of the bottom coils of the helix of Fig. 3S

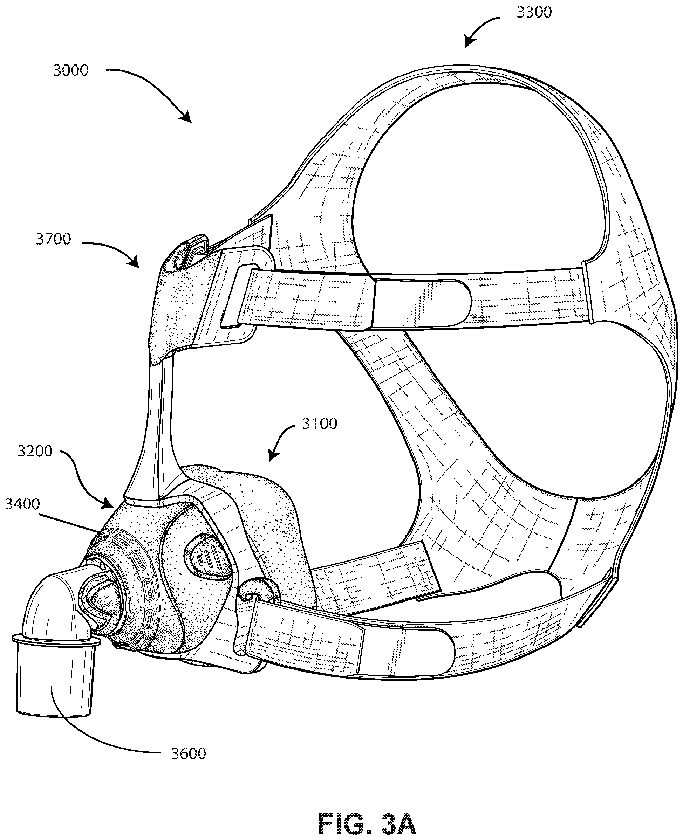

With reference to the right-hand rule of Fig. 3P, a space curve turning towards the direction of the right-hand binormal may be considered as having a right-hand positive torsion (e.g. a right-hand helix as shown in Fig. 3S). A space curve turning away from the direction of the right-hand binormal may be considered as having a right-hand negative torsion (e.g. a left-hand helix).

Equivalently, and with reference to a left-hand rule (see Fig. 3O), a space curve turning towards the direction of the left-hand binormal may be considered as having a left-hand positive torsion (e.g. a left-hand helix). Hence left-hand positive is equivalent to right-hand negative. See Fig. 3T.

### 4.9.6.4 Holes

A surface may have a one-dimensional hole, e.g. a hole bounded by a plane curve or by a space curve. Thin structures (e.g. a membrane) with a hole, may be described as having a one-dimensional hole. See for example the one dimensional hole in the surface of structure shown in Fig. 3I, bounded by a plane curve.

A structure may have a two-dimensional hole, e.g. a hole bounded by a surface. For example, an inflatable tyre has a two dimensional hole bounded by the interior surface of the tyre. In another example, a bladder with a cavity for air or gel could have a two-dimensional hole. See for example the cushion of Fig. 3L and the example cross-sections therethrough in Fig. 3M and Fig. 3N, with the interior surface bounding a two dimensional hole indicated. In a yet another example, a conduit may comprise a one-dimension hole (e.g. at its entrance or at its exit), and a two-dimension hole bounded by the inside surface of the conduit. See also the two dimensional hole through the structure shown in Fig. 3K, bounded by a surface as shown.

### 4.10 OTHER REMARKS

A portion of the disclosure of this patent document contains material which is subject to copyright protection. The copyright owner has no objection to the facsimile reproduction by anyone of the patent document or the patent disclosure, as it appears in Patent Office patent files or records, but otherwise reserves all copyright rights whatsoever.

Unless the context clearly dictates otherwise and where a range of values is provided, it is understood that each intervening value, to the tenth of the unit of the lower limit, between the upper and lower limit of that range, and any other stated or intervening value in that stated range is encompassed within the technology. The upper and lower limits of these intervening ranges, which may be independently included in the intervening ranges, are also encompassed within the technology, subject to any specifically excluded limit in the stated range. Unless the context clearly dictates otherwise, where a range of values is provided the range includes the upper and lower limits of the range (i.e. the range is inclusive). Where the stated range includes one or both of the limits, ranges excluding either or both of those included limits are also included in the technology.

Furthermore, where a value or values are stated herein as being implemented as part of the technology, it is understood that such values may be approximated, unless otherwise stated, and such values may be utilized to any suitable significant digit to the extent that a practical technical implementation may permit or require it.

Unless defined otherwise, all technical and scientific terms used herein have the same meaning as commonly understood by one of ordinary skill in the art to which this technology belongs. Although any methods and materials similar or equivalent to those described herein can also be used in the practice or testing of the present technology, a limited number of the exemplary methods and materials are described herein.

When a particular material is identified as being used to construct a component, obvious alternative materials with similar properties may be used as a substitute. Furthermore, unless specified to the contrary, any and all components herein described are understood to be capable of being manufactured and, as such, may be manufactured together or separately.

It must be noted that as used herein and in the appended claims, the singular forms "a", "an", and "the" include their plural equivalents, unless the context clearly dictates otherwise.

All publications mentioned herein are intended to disclose and describe the methods and/or materials which are the subject of those publications. The publications discussed herein are provided solely for their disclosure prior to the filing date of the present application. Nothing herein is to be construed as an admission that the present technology is not entitled to antedate such publication by virtue of prior invention. Further, the dates of publication provided may be different from the actual publication dates, which may need to be independently confirmed.

The terms "comprises" and "comprising" should be interpreted as referring to elements, components, or steps in a non-exclusive manner, indicating that the referenced elements, components, or steps may be present, or utilized, or combined with other elements, components, or steps that are not expressly referenced.

The subject headings used in the detailed description are included only for the ease of reference of the reader and should not be used to limit the subject matter found throughout the disclosure or the claims. The subject headings should not be used in construing the scope of the claims or the claim limitations.

Although the technology herein has been described with reference to particular examples, it is to be understood that these examples are merely illustrative of the principles and applications of the technology. In some instances, the terminology and symbols may imply specific details that are not required to practice the technology. For example, although the terms "first" and "second" may be used, unless otherwise specified, they are not intended to indicate any order but may be utilised to distinguish between distinct elements. Furthermore, although process steps in the methodologies may be described or illustrated in an order, such an ordering is not required. Those skilled in the art will recognize that such ordering may be modified and/or aspects thereof may be conducted concurrently or even synchronously.

It is therefore to be understood that numerous modifications may be made to the illustrative examples and that other arrangements may be devised without departing from the scope of the technology The present invention is defined by the appended claims.

### 4.11 REFERENCE SIGNS LIST

| | |
|---|---|
| patient | 1000 |
| bed partner | 1100 |
| patient interface | 3000 |
| seal forming structure | 3100 |
| plenum chamber | 3200 |
| chord | 3210 |
| superior point | 3220 |
| inferior point | 3230 |
| positioning and stabilising structure | 3300 |
| vent | 3400 |
| connection port | 3600 |
| forehead support | 3700 |
| RPT device | 4000 |
| external housing | 4010 |
| upper portion | 4012 |
| lower portion | 4014 |
| panel | 4015 |
| chassis | 4016 |
| handle | 4018 |
| pneumatic block | 4020 |
| air filter | 4110 |
| inlet air filter | 4112 |
| muffler | 4120 |
| outlet muffler | 4124 |
| pressure generator | 4140 |
| blower | 4142 |
| motor | 4144 |
| anti-spillback valve | 4160 |
| air circuit | 4170 |
| supplementary gas | 4180 |
| electrical components | 4200 |
| PCBA | 4202 |
| electrical power supply | 4210 |
| input device | 4220 |
| transducers | 4270 |
| humidifier | 5000 |
| humidifier inlet | 5002 |
| humidifier outlet | 5004 |
| humidifier base | 5006 |
| humidifier reservoir | 5110 |
| conductive portion | 5120 |
| reservoir dock | 5130 |
| locking lever | 5135 |
| water level indicator | 5150 |
| heating element | 5240 |
| connector | 6000 |
| first connector part | 6010 |
| second connector part | 6020 |
| body | 6030 |
| passage | 6032 |
| tubular inner wall | 6040 |
| spiral groove | 6050 |
| helical portion | 6050a |
| helical portion | 6050b |
| helical portion | 6050c |
| helical portion | 6050d |
| first end of tubular wall | 6052 |
| groove entrance | 6060 |
| helix angle | HA1 |
| helix angle | HA2 |
| distal end | 6070 |
| radially extending wall | 6080 |
| end of passage | 6090 |
| magnetic or ferromagnetic portion | 6100 |
| tubular outer wall | 6110 |
| passage | 6120 |
| radially projecting portion | 6130 |
| radially extending wall | 6140 |
| magnetic portion | 6150 |
| width of entrance | We |
| Distance between entrances | D |
| Width of projecting portion | Wp |
| land | 6160 |
| first body part | 6170 |
| second body part | 6180 |
| socket | 6190 |
| inner wall | 6200 |
| collar | 6210 |
| outer surface | 6220 |
| annular channel | 6230 |
| annular rib | 6240 |
| first connector part body | 6300 |
| second connector part body | 6310 |
| first connector part passage | 6320 |
| second connector part passage | 6330 |
| engaging face | 6340 |
| engaging face | 6350 |
| cylindrical outer wall | 6360 |
| arms | 6370 |
| external wall | 6380 |
| mounting portion | 6390 |
| inwardly projecting portion | 6400 |
| front edge | 6410 |
| beveled inner face | 6420 |
| frontmost portion of groove | 6430 |
| distal portion of body | 6440 |
| outer surface | 6450 |
| ribs | 6460 |

## Claims

1. A fluid connector (6000) for coupling an air circuit to a respiratory therapy system component, the connector comprising a first connector part (6010) and a second connector part (6020),
the first connector part comprising a first body (6170, 6300), a first passage (6320) extending through the first body, and a cylindrical outer wall (6360), the cylindrical outer wall comprising a groov (6050) which extends around the circumference of the first body,
the second connector part comprising a second body (6180, 6310), a second passage (6330) extending through the second body, and a plurality of arms (6370) extending from a first end of the second body, each arm comprising an inwardly extending projecting portion (6400),
wherein the arms are configured to allow the inwardly extending projecting portions to engage the groove,
wherein the groove comprises a plurality of contiguous substantially helical portions (6050a, 6050b, 6050c, 6050d) having alternating orientations, wherein the number of substantially helical portions is twice the number of arms,
wherein at least one of the first and second connector parts comprises a magnetic portion (6150) and the other of the first and second connector parts comprises a second magnetic portion and/or a ferromagnetic portion(6100), wherein magnetic attraction between the magnetic portion and the second magnetic portion and/or ferromagnetic portion biases the first and second connector parts towards a fully connected configuration, and wherein, when the first and second connector parts are in a fully connected configuration, rotation of one of the connector parts relative to the other connector part causes the first and second bodies to move away from each other.

2. The fluid connector of claim 1, wherein the first body has a first end and a second end, wherein an entrance to the first passage is provided at the first end, and wherein the magnetic attraction between the magnetic portions or the magnetic portion and the ferromagnetic portion is sufficient to move the first and second connector parts to the fully connected configuration when the inwardly extending projecting portions engage portions of the groove which are closest to the first end of the first body.

3. The fluid connector of claim 2, wherein the portions of the groove which are closest to the first end of the first body are open to the first end of the first body.

4. The fluid connector of claim 2, wherein the portions of the groove which are closest to the first end of the first body each comprise a ramped portion configured to urge the arms apart when the first connector part is disconnected from the second connector part.

5. The fluid connector of any one of claims 1 to 4, wherein each arm is connected to the external wall of the second body by a respective mounting portion, portion (6390), wherein each mounting portion comprises a resilient living hinge.

6. The fluid connector of any one of claims 1 to 5, wherein each arm is resiliently flexible.

7. The fluid connector of any one of claims 1 to 6, wherein a front edge of the first body is bevelled to urge the arms radially outward as the first and second connector parts are brought into contact.

8. The fluid connector of any one of the preceding claims, wherein the attractive force between the magnetic portions is no more than 10N when the first and second connector parts are in the fully engaged position.

9. The fluid connector of any one of the preceding claims, wherein a tension force of at least 20N is required to separate the first and second connector parts when in the fully engaged position.

10. The connector of any one of claims 1 to 9, wherein the first connector part is engaged with a patient interface (3000).

11. The connector of any one of claims 1 to 10, coupled to an air circuit. circuit (4170).

12. The connector of any one of claims 1 to 11 wherein the second connector part comprises a first body part and a second body part which is rotatable relative to the first body part.

## Patentansprüche

1. Fluidverbinder (6000) zum Koppeln eines Luftkreislaufs mit einer Komponente eines Atemtherapiesystems, wobei der Verbinder ein erstes Verbinderteil (6010) und ein zweites Verbinderteil (6020) umfasst, wobei das erste Verbinderteil einen ersten Körper (6170, 6300), einen ersten Durchgang (6320), der sich durch den ersten Körper erstreckt, und eine zylindrische Außenwand (6360) umfasst, wobei die zylindrische Außenwand eine Nut (6050) umfasst, die sich um den Umfang des ersten Körpers erstreckt, wobei das zweite Verbinderteil einen zweiten Körper (6180, 6310), einen zweiten Durchgang (6330), der sich durch den zweiten Körper erstreckt, und eine Mehrzahl von Armen (6370) umfasst, die sich von einem ersten Ende des zweiten Körpers erstrecken, wobei jeder Arm einen sich nach innen erstreckenden Vorsprung (6400) umfasst, wobei die Arme dazu konfiguriert sind, zuzulassen, dass die sich nach innen erstreckenden Vorsprünge in die Nut eingreifen,
wobei die Nut eine Mehrzahl von zusammenhängenden im Wesentlichen schraubenförmigen Abschnitten (6050a, 6050b, 6050c, 6050d) mit alternierenden Ausrichtungen umfasst, wobei die Anzahl der im Wesentlichen helikalen Abschnitte das Zweifache der Anzahl der Arme beträgt,
wobei wenigstens eines von dem ersten und dem zweiten Verbinderteil einen magnetischen Abschnitt (6150) umfasst und das andere von dem ersten und dem zweiten Verbinderteil einen zweiten magnetischen Abschnitt und/oder einen ferromagnetischen Abschnitt (6100) umfasst, wobei magnetische Anziehung zwischen dem magnetischen Abschnitt und dem zweiten magnetischen Abschnitt und/oder ferromagnetischen Abschnitt
das erste und das zweite Verbinderteil in Richtung einer vollständig verbundenen Konfiguration vorspannt, und wobei, wenn sich das erste und das zweite Verbinderteil in einer vollständig verbundenen Konfiguration befinden, eine Drehung eines der Verbinderteile relativ zu dem anderen Verbinderteil bewirkt, dass sich der erste und der zweite Körper voneinander weg bewegen.

2. Fluidverbinder nach Anspruch 1, wobei der erste Körper ein erstes Ende und ein zweites Ende aufweist, wobei ein Eingang zu dem ersten Durchgang an dem ersten Ende vorgesehen ist, und wobei die magnetische Anziehung zwischen den magnetischen Abschnitten oder dem magnetischen Abschnitt und dem ferromagnetischen Abschnitt ausreichend ist, um das erste und das zweite Verbinderteil in die vollständig verbundene Konfiguration zu bewegen, wenn die sich nach innen erstreckenden Vorsprünge in Abschnitte der Nut eingreifen, die dem ersten Ende des ersten Körpers am nächsten sind.

3. Fluidverbinder nach Anspruch 2, wobei die Abschnitte der Nut, die dem ersten Ende des ersten Körpers am nächsten sind, zu dem ersten Ende des ersten Körpers offen sind.

4. Fluidverbinder nach Anspruch 2, wobei die Abschnitte der Nut, die dem ersten Ende des ersten Körpers am nächsten sind, jeweils einen schrägen Abschnitt umfassen, der dazu konfiguriert ist, die Arme auseinanderzudrängen, wenn das erste Verbinderteil von dem zweiten Verbinderteil getrennt wird.

5. Fluidverbinder nach einem der Ansprüche 1 bis 4, wobei jeder Arm durch einen jeweiligen Montageabschnitt, Abschnitt (6390), mit der Außenwand des zweiten Körpers verbunden ist, wobei jeder Montageabschnitt ein elastisches Filmscharnier umfasst.

6. Fluidverbinder nach einem der Ansprüche 1 bis 5, wobei jeder Arm elastisch flexibel ist.

7. Fluidverbinder nach einem der Ansprüche 1 bis 6, wobei eine Vorderkante des ersten Körpers abgeschrägt ist, um die Arme radial nach außen zu drängen, während das erste und das zweite Verbinderteil miteinander in Kontakt gebracht werden.

8. Fluidverbinder nach einem der vorhergehenden Ansprüche, wobei die Anziehungskraft zwischen den magnetischen Abschnitten nicht mehr als 10N beträgt, wenn sich das erste und das zweite Verbinderteil in der vollständig in Eingriff stehenden Position befinden.

9. Fluidverbinder nach einem der vorhergehenden Ansprüche, wobei eine Zugkraft von wenigstens 20N erforderlich ist, um das erste und das zweite Verbinderteil zu trennen, wenn sie sich in der vollständig in Eingriff stehenden Position befinden.

10. Verbinder nach einem der Ansprüche 1 bis 9, wobei das erste Verbinderteil mit einer Patientenschnittstelle (3000) in Eingriff steht.

11. Verbinder nach einem der Ansprüche 1 bis 10, gekoppelt mit einem Luftkreislauf (4170).

12. Verbinder nach einem der Ansprüche 1 bis 11, wobei das zweite Verbinderteil ein erstes Körperteil und ein zweites Körperteil umfasst, das relativ zu dem ersten Körperteil drehbar ist.

## Revendications

1. Connecteur pour fluide (6000) pour raccorder un circuit d'air à un composant d'un système de thérapie respiratoire, le connecteur comprenant une première partie de connecteur (6010) et une deuxième partie de connecteur (6020), la première partie de connecteur comprenant un premier corps (6170, 6300), un premier passage (6320) s'étendant à travers le premier corps, et une paroi externe cylindrique (6360), la paroi externe cylindrique comprenant une rainure (6050) qui s'étend autour de la circonférence du premier corps, la deuxième partie de connecteur comprenant un deuxième corps (6180, 6310), un deuxième passage (6330) s'étendant à travers le deuxième corps, et plusieurs bras (6370) s'étendant depuis une première extrémité du deuxième corps, chaque bras comprenant une partie saillante (6400) s'étendant vers l'intérieur, dans lequel les bras sont conçus pour permettre aux parties saillantes s'étendant vers l'intérieur de coopérer avec la rainure, dans lequel la rainure comprend plusieurs parties sensiblement hélicoïdales contiguës (6050a, 6050b, 6050c, 6050d) ayant des orientations alternées, dans lequel le nombre de parties sensiblement hélicoïdales est deux fois le nombre de bras, dans lequel au moins l'une des première et deuxième parties de connecteur comprend une partie magnétique (6150) et l'autre des première et deuxième parties de connecteur comprend une deuxième partie magnétique et/ou une partie ferromagnétique (6100), dans lequel l'attraction magnétique entre la partie magnétique et la deuxième partie magnétique et/ou la partie ferromagnétique pousse les première et deuxième parties de connecteur vers une configuration entièrement connectée, et dans lequel, lorsque les première et deuxième parties de connecteur sont dans une configuration entièrement connectée, la rotation de l'une des parties de connecteur par rapport à l'autre partie de connecteur amène les premier et deuxième corps à s'éloigner l'un de l'autre.

2. Connecteur pour fluide selon la revendication 1, dans lequel le premier corps a une première extrémité et une deuxième extrémité, dans lequel une entrée vers le premier passage est prévue à la première extrémité, et dans lequel l'attraction magnétique entre les parties magnétiques ou entre la partie magnétique et la partie ferromagnétique est suffisante pour déplacer les première et deuxième parties de connecteur vers la configuration entièrement connectée lorsque les parties saillantes s'étendant vers l'intérieur coopèrent avec les parties de la rainure qui sont les plus proches de la première extrémité du premier corps.

3. Connecteur pour fluide selon la revendication 2, dans lequel les parties de la rainure qui sont les plus proches de la première extrémité du premier corps sont ouvertes sur la première extrémité du premier corps.

4. Connecteur pour fluide selon la revendication 2, dans lequel les parties de la rainure qui sont les plus proches de la première extrémité du premier corps comprennent chacune une partie en rampe configurée pour écarter les bras lorsque la première partie de connecteur est déconnectée de la deuxième partie de connecteur.

5. Connecteur pour fluide selon l'une quelconque des revendications 1 à 4, dans lequel chaque bras est connecté à la paroi externe du deuxième corps par une partie de montage respective, la partie (6390), dans lequel chaque partie de montage comprend une charnière élastique.

6. Connecteur pour fluide selon l'une quelconque des revendications 1 à 5, dans lequel chaque bras est flexible de manière élastique.

7. Connecteur pour fluide selon l'une quelconque des revendications 1 à 6, dans lequel un bord avant du premier corps est biseauté pour pousser les bras radialement vers l'extérieur lorsque les première et deuxième parties de connecteur sont mises en contact.

8. Connecteur pour fluide selon l'une quelconque des revendications précédentes, dans lequel la force d'attraction entre les parties magnétiques ne dépasse pas 10 N lorsque les première et deuxième parties de connecteur sont dans la position de coopération totale.

9. Connecteur pour fluide selon l'une quelconque des revendications précédentes, dans lequel une force de tension d'au moins 20 N est nécessaire pour séparer les première et deuxième parties de connecteur lorsqu'elles sont dans la position de coopération totale.

10. Connecteur selon l'une quelconque des revendications 1 à 9, dans lequel la première partie de connecteur coopère avec une interface patient (3000).

11. Connecteur selon l'une quelconque des revendications 1 à 10, couplé à un circuit d'air, le circuit (4170).

12. Connecteur selon l'une quelconque des revendications 1 à 11, dans lequel la deuxième partie de connecteur comprend une première partie de corps et une deuxième partie de corps qui peut tourner par rapport à la première partie de corps.
